# EUROPEAN PATENT APPLICATION

(11) **EP 4 527 926 A1**
(43) Date of publication of application: **26.03.2025**
(21) Application number: 23199021.9
(22) Date of filing: 22.09.2023
(51) Int. Cl.: C12N 15/11, C12N 15/86, C40B 40/06

(54) **BAC ENGINEERING WITH HALF-SITE DIRECTED FRAGMENT REPLACEMENT**

(71) Applicant: Albert-Ludwigs-Universität Freiburg, 79098 Freiburg (DE)
(72) Inventor: Ruzsics, Zsolt, 86911 Dießen am Ammersee (DE); Fischer, Julian, 79106 Freiburg (DE)
(74) Representative: Hertin und Partner Rechts- und Patentanwälte PartG mbB

(57) **Abstract**

The invention relates to a method of constructing a nucleic acid molecule of interest comprising providing a circular nucleic acid molecule comprising a cloning or target site of interest, determining the presence of partial target motifs for at least one sequence specific endonuclease flanking the cloning site of interest, and wherein the circular nucleic acid molecule does not comprise the complete target motif for the at least one sequence specific endonuclease, inserting at the cloning site of interest a nucleic acid sequence comprising a (antibiotic) resistance gene through homologous recombination, wherein the inserted (antibiotic) resistance gene is flanked (up- and downstream) by nucleic acid sequences each comprising a sequence completing the respective partial target motif of the at least one sequence specific endonuclease, digesting the modified nucleic acid molecule with the at least one sequence specific endonuclease, thereby i) deleting the (antibiotic) resistance gene from the nucleic acid molecule and ii) linearizing the nucleic acid molecule, and finally constructing the nucleic acid molecule of interest by inserting a nucleic acid sequence of interest at the cloning site of interest into the linearized nucleic acid molecule, thereby re-circularizing the nucleic acid molecule. The invention relates in another aspect to the nucleic acid molecule generated by the method of the invention and to the use of said molecules or recombinant viruses derived from them in the treatment or vaccination of a subject.

## Description

The invention lies in the field of biology, particularly in the field of microbiology, genetics and biotechnology.

The invention relates to a method of constructing a nucleic acid molecule of interest comprising providing a circular nucleic acid molecule comprising a cloning or target site of interest, determining the presence of partial target motifs for at least one sequence specific endonuclease flanking the cloning site of interest, and wherein the circular nucleic acid molecule does not comprise the complete target motif for the at least one sequence specific endonuclease, inserting at the cloning site of interest a nucleic acid sequence comprising a (antibiotic) resistance gene through homologous recombination, wherein the inserted (antibiotic) resistance gene is flanked (up- and downstream) by nucleic acid sequences each comprising a sequence completing the respective partial target motif of the at least one sequence specific endonuclease, digesting the modified nucleic acid molecule with the at least one sequence specific endonuclease, thereby i) deleting the (antibiotic) resistance gene from the nucleic acid molecule and ii) linearizing the nucleic acid molecule, and finally constructing the nucleic acid molecule of interest by inserting a nucleic acid sequence of interest at the cloning site of interest into the linearized nucleic acid molecule, thereby re-circularizing the nucleic acid molecule. The invention relates in another aspect to the nucleic acid molecule generated by the method of the invention and to the use of said molecules or recombinant viruses derived from them in the treatment or vaccination of a subject.

### BACKGROUND OF THE INVENTION

Adenoviruses are linear double stranded DNA viruses, which replicate efficiently in cell culture. Recombinant adenovirus (rAd) vectors promise wide applicability as gene transfer vehicles for both in vivo and in vitro applications. Worldwide, 14 different *in vivo* viral gene therapies are currently approved^{1,2}. Out of those, seven are based on human adenoviruses and another one on chimpanzee adenovirus. Outside of this, numerous clinical trials are running using recombinant adenovirus (rAd) vectors as well as even more research applications utilize rAd-based vectors.

However, when taking a closer look at currently ongoing clinical trials, rAd-based vectors loose their prime position to recombinant adeno-associated viruses (rAAV). Recombinant adenoviruses have been the preferred vector system for gene and cell therapy approaches in humans, as well as for immunizations. However, other systems, such as adeno-associated viruses or lentiviruses have caught up. This is mainly due to the disadvantages of adenovirus systems, such as limited targeting efficiency, immunogenicity, potential toxicity, limited duration of gene expression.

In general, rAds are an attractive platform for gene transfer applications due to their large capacity for transgenes, simple cloning- and production-principle as well as their ability to mediate efficient gene transfer both *in vitro* and *in vivo.* rAd genomes have been constructed in E. *coli* where their genomes can be maintained, propagated and modified in a form of circular plasmid or bacterial artificial chromosome (BACs). However, during recent years, rAAV vector technology allowed for randomized recombination of viral serotypes, generating a huge amount of novel recombinant capsids³. Using these feature libraries paved the way for screening for both novel tropism variants, allowing for the targeting of specific tissue or de-targeting to remove unwanted off-target effects⁴⁻⁶.

In order to allow library reconstitution, a sufficiently effective method is required to receive any notable amount of virus from diversified genetic material. For rAAV, this method simply consists of co-transfection of plasmids into producer cells with helper plasmids, which are required for production. Recently, the present inventors proposed a novel method to rescue rAd libraries with efficiencies comparable to those of AAV production. This technology is based on CRISPR/Cas mediated terminal resolution (CTR), which allows high frequency of rAd rescue in different cell types. However, while AAV vector genomes can be maintained in simple high copy plasmids in E. *coli* and can be modified with high efficiencies, modification of rAd genome rarely reaches library efficiencies, matching the diversity of 10^4 - 10^5 individual clones, which can be theoretically reached by CTR.

Due to the success of rAds as vector platform, modification of their genomes by either inserting transgenes or manipulating the viral genes is well established. One of these methodologies is the use of homologous recombination as a method of modifying nucleotides at specific loci using and inserting linear fragments into the viral genome based on chosen homologies using BAC technology in E. *coli* ³⁵ . Another way is the use of Gibson assembly of a set of genomic fragments either generated by pre-existing restriction sites, or after inserting a complete unique restriction site, usually using meganucleases, to linearize the vector to receive a PCR- or plasmid derived insert^{36,37}.

With present classical DNA engineering methods such as restriction enzyme-based cloning, mutagenesis via PCR, recombination technologies, recombinant adenovirus genomes can be modified only in limited scale. The currently available methods are largely inefficient and not suitable for the production of large viral DNA libraries. Such methods are suitable for construction of single recombinants but suffer from either low efficiency due to the assembly of multiple fragments, or lack flexibility due to the fixed position of the unique restriction site. Therefore, the above technologies did not match the efficiency of the recently published rAd rescue platform called CTR (allowing about 10^4 to 10^5 independent rescues for one transfection³⁸) limiting the size of possible diversity of potential adenovirus-based libraries with orders of magnitude.

In light of the prior art there remains a significant need in the art to provide improved means and methods for the reliable and flexible modification of larger bacterial vectors, e.g., bacterial chromosomes (BAC), such as those comprising infectious rAd-genomes.

### SUMMARY OF THE INVENTION

In light of the prior art the technical problem underlying the present invention is the provision of improved means and methods for the reliable and flexible generation of recombinant (adeno)viral libraries and/or larger bacterial vectors, with a higher flexibility regarding genetic modifications, insertion sites and an improved efficiency over prior art methods, e.g., present BAC library methods.

This problem is solved by the features of the independent claims. Preferred embodiments of the present invention are provided by the dependent claims.

In one aspect the present method relates to a method of constructing a nucleic acid molecule of interest comprising
a. Providing a circular nucleic acid molecule comprising a cloning site of interest,
b. Determining the presence of partial target motifs for at least one sequence specific endonuclease flanking the cloning site of interest (up- and downstream thereof), wherein said partial target motifs respectively comprise only a fraction (e.g., between 20-80 %), preferably essentially half (e.g., between 40-60 %, or about 50%), of the nucleic acid sequence of the complete target motif for the at least one sequence specific endonuclease, and wherein the circular nucleic acid molecule does not comprise the complete target motif for the at least one sequence specific endonuclease,
c. Modifying the nucleic acid molecule by inserting at the cloning site of interest a nucleic acid sequence comprising a (antibiotic) resistance gene through homologous recombination, wherein the inserted (antibiotic) resistance gene is flanked (up- and down-stream) by nucleic acid sequences each comprising the a sequence completing the respective partial target motif missing elements of the complete target motif of the at least one sequence specific endonuclease determined in step b., thereby generating a modified nucleic acid molecule comprising two complete target motifs of the at least one sequence specific endonuclease,
d. Optionally selecting/amplifying the nucleic acid molecule modified in step c. in a micro-organism, wherein the microorganism (comprising the nucleic acid molecule) is cultivated in the presence of the (antibiotic) substance corresponding to the (antibiotic) resistance gene inserted into the nucleic acid molecule in step c.,
e. Digesting the modified nucleic acid molecule with the at least one sequence specific endonuclease, thereby i) deleting the (antibiotic) resistance gene from the nucleic acid molecule and ii) linearizing the nucleic acid molecule,
f. Constructing the nucleic acid molecule of interest by inserting a nucleic acid sequence of interest,
   preferably together with nucleic acid sequences up- and/or downstream (proximal) of the cloning site of interest deleted in step c.,
   into the nucleic acid molecule linearized in step e., preferably using an in vitro fragment assembly technique, more preferably using Gibson assembly, thereby re-circularizing the nucleic acid molecule, and preferably restoring the sequence of the partial target motifs, and optionally also further nucleic acid sequence regions up- and/or downstream (proximal) of the partial target motifs and/or the cloning site of interest, determined in step b.

In embodiments the nucleic acid is DNA. In embodiments the circular nucleic acid molecule is a circular DNA molecule. In embodiments the sequence of interest is selected from (synthetic) linear DNA, a PCR fragment, a cDNA sequence generated from gRNA and a linearized cloning vector.

In embodiments the circular nucleic acid molecule is a circular DNA molecule. In embodiments the circular nucleic acid molecule is a circular double stranded DNA molecule.

In embodiments, the in step f. re-circularized nucleic acid molecules are re-introduced into bacteria for further amplification and/or may be used for rescuing recombinant viruses or libraries of recombinant viruses.

Therefore, in embodiments the present method further comprises step g. introducing the in step f. re-circularized nucleic acid molecules into bacteria and amplifying the nucleic acid molecules in said bacteria, and/or using the nucleic acid molecules for rescuing recombinant viruses or libraries of recombinant viruses.

In embodiments herein the partial target motifs for at least one sequence specific endonuclease flanking the cloning site of interest in the circular nucleic acid molecule may be flanked by nucleic acid sequences similar, identical and/or homologous to those flanking an insert comprising the (antibiotic) resistance gene, such that homologous recombination can occur between the cloning site of interest and the sequence to be inserted by homologous recombination in the context of the present method, e.g., in step c., as described herein. In embodiments two similar, identical and/or homologous nucleic acid sequences may have a length of between 4-300, preferably between 4-100 nucleotides/bases and share a sequence identity allowing homologous recombination, such as e.g., a sequence identity of between 50-100%, of between 60-100 %, or of 50%, 55%, 60%, 65%, 70%, 75%, 80%, 85%, 90%, 95, 99% or even 100%. In embodiments the insert introduced into the circular nucleic acid e.g., in step c., comprising the (antibiotic) resistance gene is specifically designed to comprise sequences flanking the (antibiotic) resistance gene that are similar, identical and/or homologous to those flanking the partial target motifs within the circular nucleic acid molecule, such that the (antibiotic) resistance gene may be introduced, e.g., in step c, into the circular nucleic acid molecule by homologous recombination at the cloning site of interest.

In embodiments the circular nucleic acid molecule, preferably a circular DNA molecule, is a bacterial chromosome. In embodiments the circular nucleic acid (e.g., DNA) molecule is a bacterial artificial chromosome (BAC).

In some embodiments the BAC comprises a (recombinant) adenoviral (rAd) genome or part thereof or an rAd vector. In some embodiments the sequence of interest is or comprises a (recombinant) adenoviral (rAd) genome or part thereof or an rAd vector, which is integrated into the circular DNA molecule, e.g., a (much larger) BAC. Using BAC-based circular DNA molecules is advantageous due to the large cloning capacity of BACs which enables for example delivery of DNA sequences, e.g., partial or entire rAd genomes, enabling expression of the sequence of interest.

In embodiments, a circular nucleic acid molecule, e.g., as used in step a., comprises a BAC comprising a (recombinant) adenoviral (rAd) genome or part thereof. In other words, in embodiments, a circular nucleic acid molecule comprises a BAC and a (recombinant) adenoviral (rAd) genome or part thereof. In some embodiments wherein a circular nucleic acid molecule comprises a BAC and a (recombinant) adenoviral (rAd) genome or part thereof, the sequence of interest comprises or consist of a (adeno)viral nucleic acid sequence and/or another sequence of interest, e.g., a nucleic acid sequence encoding an antibody (or fragment thereof) and/or any other nucleic acid sequence intended to be inserted into the circular nucleic acid molecule (e.g., a nucleic acid sequence intended for gene therapy etc.).

In embodiments the sequence of interest is or comprises a partially or fully infectious recombinant adenovirus (rAd)-genome. In embodiments the circular nucleic acid in step a. and/or in step f. comprises a partially or fully infectious recombinant adenovirus (rAd)-genome. In embodiments the sequence of interest is or comprises one or more genes of a (recombinant) adenovirus. In embodiments the adenoviral genome is a human adenoviral vector genome. In embodiments the adenovirus is a human adenovirus. In embodiments the sequence of interest comprises one or more mutations in comparison to a wt (recombinant) adenovirus or a host (e.g., human, or other mammalian) genome.

In embodiments the sequence of interest comprises or consists of the nucleic acid sequence of a gene or part thereof. In embodiments the sequence of interest comprises or consists of a gene carrying a mutation in comparison to a wildtype sequence. In embodiments the sequence of interest comprises or consists of a gene carrying a wildtype sequence or (`healthy') variant of a gene, e.g., a gene known to have an undesired or disadvantageous mutation. In embodiment an undesired or disadvantageous mutation may be linked or associated with a certain disease, condition or phenotype. In embodiments the sequence of interest comprises or consists of a gene not comprising a mutation and/or associated with a favorable, e.g., healthy, phenotype.

In embodiments the sequence of interest has a length of between 1-7500 nt (nucleotides), or of between 4-3500 nt, or of between 5-4000 or between 5-1000, 10-2000, 15-3000 nt. The inventors could show that the method is suitable for sequences of interest such as, e.g., synthetic dsDNA fragments or PCR fragments or even sub-cloned fragments, if they can be seamlessly released from their plasmid form, which may be integrated as sequence of interest.

In an exemplary, non-limiting, example of the present method a sequence of interest comprises a transgene. In some of said exemplary, non-limiting, examples the capacity (for inserted nucleic acids) of a circular nucleic acid, which may e.g., be an rAd vector or a BAC comprising a rAd genome or part thereof, is about 7500 base pairs. In some of said exemplary, non-limiting, examples a sequence of interest comprises a sequence (expression cassette) encoding an antibody or fragment thereof, wherein in embodiments such an antibody expression cassette comprises between 6000-7500 base pairs. In some embodiments, a sequence of interest may be of about 15-3000 nt in length (comprising about 15-3000 bp).

In some preferred embodiments the circular nucleic acid (e.g., DNA) molecule is a bacterial artificial chromosome (BAC) comprising a partial or fully infectious viral genome, such as a (recombinant) adenoviral (rAd) genome. In some preferred embodiments the nucleic acid molecule of interest comprises a partial or fully infectious viral genome, such as a (recombinant) adenoviral (rAd) genome.

In embodiments the present method enables the introduction of entire genes or partial gene sequences, e.g., comprising gene variants or mutations, into a (recombinant) adenoviral (rAd) genome, wherein the introduced genes are, e.g., viral, bacterial or mammalian, such as human, genes. In embodiment the present method allows for the construction and/or modification of partially or fully infectious viruses (or viral genomes), which may subsequently be used, for example, for vaccinating or gene therapy of a subject.

In embodiments the circular DNA molecule is a high copy plasmid. Such embodiments are highly advantageous for high efficiency applications requiring high quality of DNA and introduction multiple copies of the circular DNA molecule into the producing cells (e.g., microorganisms). In such embodiments employing circular high copy plasmids comprising the recombinant adenoviral genome, it can be preferred to provide a gRNA and Cas9 or another RNA-guided endonuclease encoding sequences either on different DNA molecules, such as other plasmids, or to use producing cells lines already expressing these molecules.

In embodiments, the `cloning site of interest' may also be referred to as 'target site of interest'. The terms `cloning site of interest' and 'target site of interest' may herein preferably be used interchangeably.

In embodiments, the present method may be used to mutate (to insert mutation(s) into) the sequence of the circular nucleic acid molecule itself, e.g., the (adeno)viral sequences. In some embodiments the term 'cloning site of interest' may be referred to as 'target site of interest'. In embodiments, in step c. of the present method a resistance marker/gene is inserted into the circular nucleic acid. In some embodiments the resistance gene is then replaced/deleted in step e. and a nucleic acid sequence of interest is inserted in step f., which is in embodiments a 'foreign' sequence and/or a modified (adeno)viral sequence (e.g., comprising one or more mutations). Hence, in some embodiments the circular nucleic acid comprises (adeno)viral sequences or a (adeno)viral genome, wherein the method of the present invention is used to introduce one or more mutations into said (adeno)viral sequence/genome.

In embodiments, the in step f. re-circularized nucleic acid molecules can be re-introduced in bacteria for further amplification and/or may be used for rescuing recombinant viruses or libraries of recombinant viruses.

Using CRISPR/Cas-technology to modify viruses is one prior art method used for creating mutants, but the results are i) never clonal, and ii) always contaminated with the wild type genomes. Any of these two kinds of genetic impurity may be acceptable in research (if the null phenotype dominates), but unacceptable in any kind of vector propagation technology in which clonal and pure preparations are needed. CRISPR/Cas technology has als been suggested for linearization of circular DNA, such as plasmid DNA, in vitro (Jia-Wang Wang et al: "CRISPR/Cas9 nuclease cleavage combined with Gibson assembly for seamless cloning", Biotechniques, vol. 58, no. 4, 1 April 2015), however, such in vitro approaches are not feasible to assist rescue/transfer of rAds, e.g., as known restriction enzyme-based *in vitro* linearization techniques are relatively inefficient. The present approach provides a significant improvement over such prior art approaches, as it achieves a high efficiency and at the same time avoids the scarring of viral genomes by restrictions sites or the introduction of unwanted mutations during the engineering process.

In embodiments from the generated nucleic acid molecule of interest, e.g., comprising recombinant (adenoviral) viral genomes, said rAds may be afterwards rescued to rAd libraries using the inventors previously described CTR method (EP21778512.0 and WO2022/069523).

In embodiments, a 'target motif' of an endonucleases/restriction enzyme may refer to the recognition motif or recognition site of an endonucleases/restriction enzyme (the (specific and/or minimal) nucleic acid sequence recognized and/or bound and/or cut by an endonuclease/restriction enzyme). In embodiments, the terms 'target motif', 'target sequence', `recognition motif', `recognition sequence' and/or`recognition site' may be used interchangeably herein.

In embodiments, the present method utilizes the presence of partial target motifs ('half-sites') of endonucleases/restriction enzymes in a circular nucleic acid molecule, e.g., a genomic BACmids used for rAd-modification, wherein the endonucleases commonly do not cut within the circular nucleic acid molecule. In embodiments these half-sites proximal up- and downstream to the gene or insertion of interest are in a first step completed to allow linearization with the intended restriction enzyme, while preferably also replacing all sequences between these designated sites with an antibiotic resistance gene, allowing for positive selection for the intermediate construct. In embodiments these resistant intermediates are then, in a second step, linearized by the given restriction endonuclease and both the removed genomic sequences and the gene of interest or target mutation(s) are (re-)inserted using an assembly technique, such as Gibson assembly, based on homologous regions located adjacent to the half-sites. Depending on the application, type of inserted DNA can in embodiments also be chosen freely between, e.g., synthetic linear DNA, PCR fragments or linearized cloning vectors, with the latter providing the highest efficiency both in terms of individual clones and purity.

In order to allow, on one hand, efficient, highly reliable and on the other hand completely flexible construction of modified rAd-genomes at library efficiency, the inventors combined BAC recombineering with in vitro fragment assembly technique (e.g., Gibson assembly or similar), for the insertion of the modifications or transgenes at the targeted site to generate targeted genomes. Using the present method, the inventors were surprisingly able to achieve a 99% reliability, while also providing cloning efficiencies reaching 10^5 to 10^6 primary clones for single experiments. At the same time, due to the need to identify only fractions of sequence specific endonuclease target motifs, the present method provides nearly complete freedom in terms of selecting the position of genetic engineering. The inventors surprisingly found that the present method facilitates the reliable modification of large BACmids containing full infectious rAd-genomes at any given position with efficiencies greatly surpassing currently established library applications. In an exemplary experiment the inventors showed the practical applicability of CRISPR/Cas mediated terminal resolution (CTR) in the context of the present method for transferring said highly diverse libraries into viral vector libraries.

The present method enables `seamless' insertions and can adopt any type of insertions, e.g., from synthetic oligonucleotides to large double stranded DNA fragments. In embodiments the present two step method utilizes the presence of short, e.g., 4 nt long, sequences in the genomic BACmids used for rAd-modification, representing the 'half-sites' (fraction of target motif) of sequence specific endonucleases, which commonly do not cut within the used constructs (e.g., Swal for HAdV-C5). In one aspect of the invention the flexible selection of the partial target motifs of endonucleases in a circular nucleic acid, such as an adenoviral genome, is a great advantage over prior art methods, as the presence of complete minimal target motifs for endonucleases are not a prerequisite for the successful insertion of a sequence of interest, such that the abundant presence of such half sites (partial target motifs) in a circular nucleic acid, such as an adenovirus genome, allow basically complete coverage of the viral DNA regarding the insertion of one or more sequences of interest (and thereby a complete freedom of choice regarding the insertion/cloning site). Moreover, the inventive combination of method steps described herein facilitates in preferred embodiments the insertion of a sequence of interest without the modification of the initial circular nucleic acid, e.g., adenoviral genome, besides the sequence of interest, as, for example, neither complete target motifs of restriction enzymes are left behind (`restriction scars') in the final obtained nucleic acid molecule, e.g., modified adenoviral genome, nor are the sequences flanking the partial restriction sites in embodiments modified or lost, such that in embodiments an adenoviral genome may only be modified/changed by the insertion of the sequence of interest itself, without further modifications of the adenoviral genome sequence, e.g., unwanted mutations. Moreover, with the present method the generation of complex and large adenoviral libraries is possible, such that the disadvantages of the prior art, such as a low complexity of virus library production and side effects, such as scars or mutations after the insertion of a sequence of interest, are overcome. Current prior art DNA manipulation methods, such as restriction enzyme-based cloning, mutagenesis via PCR, recombination technologies facilitate only the modification of recombinant adenovirus genomes in small numbers and are highly inefficient and therefore not suitable for the production of large virus DNA libraries.

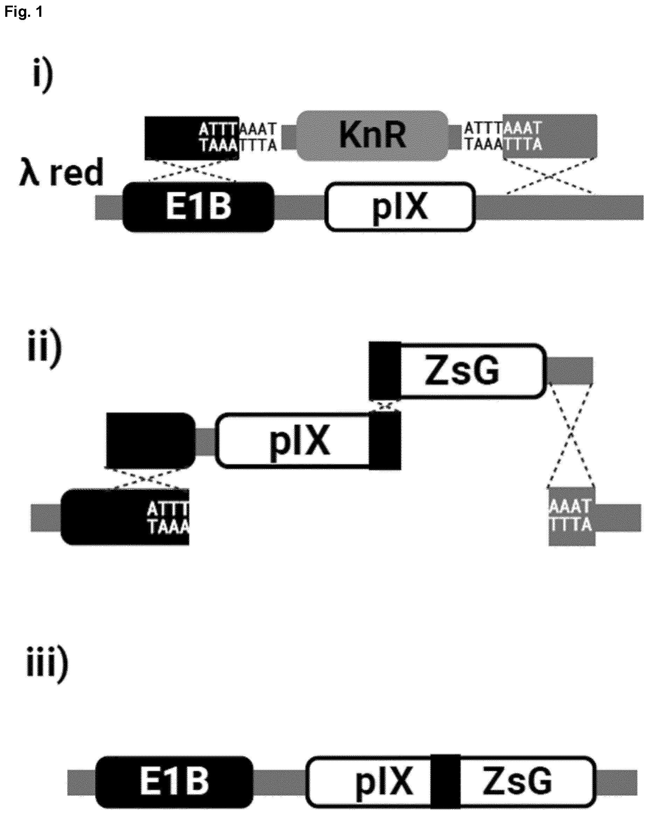

In embodiments said 'half-sites' that are (proximal) up- and downstream to the gene or insertion of interest are completed to the full length target motif in a first step, which preferably allows linearization with the intended one or more sequence specific endonuclease(s). In embodiments, at the same time, all sequences between these designated sites (motifs) are replaced with an antibiotic resistance gene, allowing for positive selection for the intermediate construct. In embodiments these double-resistant intermediates are then, in a second step, linearized by the given restriction endonuclease(s) and both the removed genomic sequences and the gene of interest or target mutation(s) are re-inserted using, e.g., in vitro fragment assembly techniques such as Gibson assembly, based on 'half-site- adjacent' homologous regions (see, for example, graphical abstract in Figure 1). Depending on the application, in embodiments the type of inserted DNA can also be chosen freely, e.g., synthetic linear DNA, PCR fragments and linearized cloning vectors with the latter preferably providing the highest efficiency both in terms of individual clones and purity. During testing with proof-of-principle libraries, the inventors could show that their new methodology was able to achieve diversities >10^6 individual and unique clones based on 10 ng of BACmid. With a correct assembly of >99% of genomes, the present method surprisingly improves currently established methods⁵ at least 10-fold, while also allowing more freedom in choosing insertion sites and therefore modifying not only the transgenes bot also the vector with high efficiency. Using the present methodology to reconstitute recombinant viruses then allowed the inventors to rescue these diversified libraries with an efficiency of ~2.5×10^4 per cm^2 of transfected cell culture. This new freedom furthermore expands the scope of rAd-library applications, for example, from just highly diverse transgene carriers (e.g., gRNA libraries) to the generation of vector libraries for optimization of their performance. In embodiments herein the present method may also be referred to as half-site-directed fragment replacement (HFR).

In embodiments a sequence specific endonuclease may be an endonuclease, a restriction endonucleases or restriction enzyme. In embodiments herein said terms may be used interchangeably.

In embodiments step b. comprises determining the presence of partial target motifs for two different sequence specific endonucleases (such that the at least one sequence specific endonuclease comprises in embodiments two different sequence specific endonucleases), such that the cloning site of interest is flanked by partial target motifs of two different endonucleases and during the method, e.g., in step e., the circular nucleic acid molecule is cut/digested upstream of the cloning site of interest by one endonuclease und downstream by the other endonuclease. Accordingly in step f., preferably the two different partial target motifs of the two different sequence specific endonucleases are restored respectively.

In embodiments the partial target motifs of the endonucleases are surrounded and/or flanked up- and/or downstream by so termed proximal (nucleic acid) sequence regions. In embodiments the proximal (nucleic acid) sequence regions are completely or partially removed by the modification of the nucleic acid molecule in step c. and optionally restored (together with the partial target motifs) in step f., such that in these embodiments the circular nucleic acid molecule comprises no sequence modifications other than the sequence of interest after step f. in comparison to the initial circular nucleic acid molecule in step a. In such embodiments the method according to the invention enables, for example, a `seamless' insertion of a nucleic acid sequence of interest. In embodiment the partial target motifs (half sites) are comprised of a 'forward' facing half site and a backwards (reverse) facing half site within the circular nucleic acid molecule.

In embodiments, in step f. inserting a nucleic acid sequence of interest into the nucleic acid molecule linearized in step e. is preferably achieved/directed by homologies (between the nucleic acid of interest) to the released (free) ends of the linearized `circular' nucleic acid, preferably containing the original (as selected in step a.) partial recognition motifs, preferably using an in vitro fragment assembly technique.

Herein "proximal" or "proximity" preferably refers to a location / sequence distance within in the range of 0 - 3000, or between 150-250 nucleotides/bases besides (up- or downstream of) the respective reference point in a nucleic acid sequence. In embodiments 'proximity' refers to a distance, such as e.g., a distance between the (two) partial target sequence motifs (recognition motifs) of endonucleases and/or a distance between a partial target motif and a cloning site of interest, such as a distance of between 1-3000, between 100-3000, between 100-500, between 500-1500, between 100-2000, between 100-1000, between 50-500 or of about 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, 50, 55, 60, 65, 70, 75, 80, 85, 90, 95, 100, 150, 200, 250, 300, 350, 400, 450, 500, 550, 600, 650, 700, 750, 800, 850, 900, 950, 1000, 1500, 2000, 2500, 3000, 3500, 4000, 4500, 5000 bases/nucleotides (nt), or any number in between.

In embodiments it is preferred, that the distance between the partial target motifs (within the circular nucleic acid molecule in step a.) differs not extensively (e.g., between +/-50bp, +/-100bp or +/- 250 bp, or +/- 500 bp) from the length of the nucleic acid sequence of interest that is to be inserted in step f. In embodiments, the (two) partial target sequence motifs are within less than 3000 nt/base pairs, less than 2500, less than 2000, less than 1500, less than 1000, less than 500, less than 300 nt (distance) from each other (within the circular nucleic acid molecule). In some embodiments the partial target sequence motifs are within a distance of between about 300 and 500 nt from each other. In some embodiments a distance of less than 1000 nt between the partial target sequence motifs is preferred, as this represents a suitable size for some nucleic acid sequences of interest, such as e.g., PCR amplicons or synthetic nucleic acid fragments. In some embodiments a distance of about 500 nt between the partial target sequence motifs is applicable for sub-cloning, however, in some embodiments a distance of about 500-1500 between the partial target sequence motifs is a more suitable distance for sub-cloning. In some embodiments it is not of interest, if the distance between the (two) partial target motifs differs from the length of the sequence of interest.

In embodiments to create complete endonuclease target motifs (recognition motifs) on the basis of partial target motifs (`half sites'; nucleic acid motifs that comprise only a fraction of the nucleic acid sequence of the complete target motif for the endonuclease) it is preferred that one forward and one reverse sequence motif is selected. In embodiments two complete target sequence motifs are generated, preferably one at the 5' of the cloning site of interest (the intended site of mutagenesis) and the other at the 3' of the cloning site of interest.

In embodiments the fraction of the nucleic acid target motif (partial target motif) of the (sequence specific) endonuclease comprises between 1-99 %, between 5-95 %, between 10-90 %, between 20-80 %, between 30-70 % between 40-60% or preferably essentially half, e.g., between 40-60 %, or about 1%, 2.5%, 5%, 10 %, 15%, 20%, 25%, 30%, 35%, 40%, 45%, 50%, 55%, 65%, 70%, 75%, 80%, 85%, 90%, 95, or even 99% of the nucleic acid sequence of the complete target motif for the endonuclease.

In embodiments a partial nucleic acid target motif (a fraction of the nucleic acid target motif) of an endonuclease has a length of between 1-30 base pairs (bp), preferably between 1-15, or between 1-8 or 1-5 bp, or of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30 or even more base pairs. In some preferred embodiments between 1-10 bp.

Accordingly, a complete nucleic acid target motif of an endonuclease has a length of between 2-50 base pairs (bp), preferably between 2-30, or between 2-20 or 1-16 bp, or of 2, 3, 4, 5, 6, 7, 8, 9, 10, 11, 12, 13, 14, 15, 16, 17, 18, 19, 20, 21, 22, 23, 24, 25, 26, 27, 28, 29, 30 or even more base pairs. In some preferred embodiments between 2-10 or of 8 bp.

In embodiments `essentially half' may in embodiments refer to a fraction, or to a fraction of about 50%, of between 40-60%, or even of between 30-70%. Essentially half of a nucleic acid sequence motif may refer in embodiments to a nucleic acid sequence comprising between 30-70%, or between 40-60%, or of about 50% of a sequence motif referred to (e.g., a 'complete' target sequence motif of an endonuclease).

Herein a 'complete' target motif of an (sequence specific) endonuclease or restriction enzyme preferably refers to a nucleic acid sequence motif that enables the recognition, binding and/or cutting (digestion) of a nucleic acid strand by an endonuclease or restriction enzyme. 'Complete' refers to a nucleic acid sequence, preferably about 100% of the minimum required sequence, which enables the desired effect achieved by the respective endonuclease or restriction enzyme, which is preferably the digestion/cutting of the nucleic acid strand by said endonuclease or restriction enzyme. In other words, the 'complete' target nucleic acid motif preferably comprises at least the minimum/minimal target nucleic acid sequence (consecutive sequence of nucleotides) of the respective endonuclease or restriction enzyme that is required for a recognition and/or cutting by said enzyme. The skilled person knows how to determine and design a 'complete' target motif of an endonuclease or restriction enzyme (e.g., cutting site).

In embodiments the endonuclease or restriction enzyme is a sequence specific endonuclease or restriction enzyme, preferably a sequence specific DNA endonuclease or restriction enzyme.

In general, numbers or factions given in percent (%) herein, may in embodiments comprise a variation of more than +/- 10%, of +/- 10%, of +/- 5 %, or of less than +/- 5%.

In embodiments the *in vitro* fragment assembly technique is a Gibson assembly. However, there are growing number of *in vitro* fragment assembly techniques, similar or comparable to Gibson assembly, presently on the market, which are claimed to be unique, while, however, functioning according to the same basic principle, namely the induction of unification of two or more fragments in a sequence (homology) directed in vitro reaction. The skilled person generally is aware of *in vitro* fragment assembly techniques comparable to Gibson assembly. One alternative may in embodiment be successive hybridization assembling (SHA).

In embodiments the circular nucleic acid molecule in step a. and/or the inserted nucleic acid molecule of interest is step f. is/are recombinant adenovirus (rAd) vector(s) or adenoviral genome(s), respectively.

In embodiments the cloning site of interest of the circular nucleic acid molecule in step a. comprises a sequence to be deleted, and where-in the sequence to be deleted is in step c. deleted by inserting a nucleic acid sequence comprising a (antibiotic) resistance into the at the cloning site of interest.

In embodiments the sequence of interest comprises at least one gene of interest, e.g., a gene comprising a mutation of interest or a therapeutic gene.

In embodiments the sequence of interest comprises a gene encoding a label or dye, such as e.g., a fluorescent protein, e.g., GFP, YFP, mCherry, mVenus, mEGFP or similar.

In embodiments the nucleic acid sequence of the complete target motif for the at least one sequence specific endonuclease has a length of between 4-14 nucleotides, preferably of about 8 nucleotides, and wherein the target motif(s) comprising essentially half of the nucleic acid sequence of the target motif for the at least one sequence specific endonuclease have a length of between 2-8 nucleotides, preferably of about 4 nucleotides.

In embodiments the sequence of interest comprises a nucleic acid sequence encoding a viral capsid or envelope, or fragments or com-ponents thereof, such as one or more proteins of a viral capsid or envelope.

In embodiments wherein the sequence of interest comprises a nucleic acid sequence encoding an antibody or fragment thereof.

In embodiments the sequence of interest comprises a nucleic acid sequence encoding a Cas9 nuclease and/or cDNA encoding gRNA.

In embodiments the final nucleic acid molecule of interest (e.g., circular DNA molecule) comprises one or more expression cassettes for one or more gRNAs and/or for Cas9. In some of such embodiments the recombinant adenoviral genome is comprised by a circular DNA molecule (e.g., BAC, since a BAC is large enough), wherein the circular DNA molecule additionally harbors the expression cassettes for one or two or more gRNA and/or Cas9 or another suitable RNA-guided endonuclease.

In embodiments of the invention, the circular nucleic acid molecule is a bacterial artificial chromosome (BAC). In such embodiments, the recombinant adenoviral genome is integrated into a much larger BAC. Using such BAC based circular DNA molecules is advantageous due to the large cloning capacity of BACs. In embodiments this enables the delivery of DNA sequences enabling expression of one, two or more gRNAs and/or Cas9 or another suitable RNA-guided endonuclease within the same DNA molecule.

In another aspect the present invention relates to a circular nucleic acid molecule constructed according to the method according to the invention.

In embodiments of the circular nucleic acid molecule according to the invention the circular nucleic acid molecule is a recombinant adenovirus (rAd) vector.

In another aspect the present invention relates to the use of the circular nucleic acid molecule according to the invention in a high-throughput screening approach.

In embodiments of the circular nucleic acid molecule the high-throughput screening approach comprises a screening of rAd-based gRNA libraries, a gene-expression-based screening of (monoclonal) antibodies, an in vitro evolution-based screening of viral particles (e.g., for immune escape- or tropism variants), enzyme variants for in vitro evolution (e.g., by screening for improved or modified activities), random poly- or oligopeptides (e.g., for screening their inhibitory effect on cellular functions), DNA sequence libraries for selecting artificial (tissue/tumor specific) enhancer elements, or randomized siRNA libraries.

In embodiments, the present nucleic acid molecule may be used in a gene-expression-based screening of (monoclonal) antibodies or parts thereof, e.g., as alternative to hybridomas-based screenings, e.g., using he rAd based gene expression in suitable cells, such as 293 cells.

In embodiments, the present nucleic acid molecule may be used in the treatment or vaccination of a subject.

In embodiments, recombinant viruses derived from the present nucleic acid molecule may be used in the treatment or vaccination of a subject.

In embodiments, the treatment is a gene therapy-based treatment.

In another aspect the present invention relates to a pharmaceutical composition comprising the circular nucleic acid molecule according to the invention for use in the treatment of a genetic disease in a subject, wherein the treatment is a gene therapy-based treatment.

In another aspect the present invention relates to an immunogenic composition, such as a vaccine, comprising the circular nucleic acid molecule according to the invention.

In embodiments the immunogenic composition according to the present invention is used for in the induction of an immune response (vaccination reaction) in a subject.

In embodiments the present method may be used for the construction of first generation rAd vectors for gene transfer applications. The present method also provides advantages of the prior art as until today the generation of single mutants has been a multi-step process with greatly limited efficiency. In addition, rAds are among the most commonly used vectors in experimental gene transfer, their application *in vivo* however is limited by either existing immunity against the vector or their inability to infect specific tissues to deliver their transgene.

To modify multiple viral proteins at different sites, which mediate these vector features, it requires a genetic workflow, which is not restricted to pre-defined regions for gene insertions. Using embodiments of the present method can allow high-throughput modification of viral capsid into random variants, which allows *in vitro* evolution-based screening, e.g., for immune escape- or tropism variants using modified rAd particles directly. Similar results were previously attempted via phage-display engineered libraries, where the targeting peptides were identified applying selection on the phage particles. However, their performance in the rAd context was notoriously low as phage- selected peptides are not evolved to fit into the rAd capsid structure⁴⁰.

Embodiments of the present method can also be used to augment existing library applications, in which expression diverse protein variants are screened. For example, hybridoma-based screening for monoclonal antibodies is a time consuming and labor-intensive process. Using embodiments of the present method as a rAd-based cloning system, can allow combining expression and screening for binding in the same format but with higher efficiency.

Moreover, embodiments of the present method may be used for direct cloning of rAd-based antibody expression vector libraries from patient-derived PBMCs, which could overcome the necessity of hybridoma propagation. Similar techniques have already been established and used, however usually as a 2-step workflow, once again relying on phage-display^{7,8} to allow for screening before cloning into rAd-based expression vectors. The direct cloning into rAd-based vectors can have the advantage that the bacterial expression of the binding domains native antibodies, which is required for the phage display, will not limit their folding and therefore the direct mammalian expression could largely increase the number of the potential hits^{41, 43}.

In addition, currently existing viral vector libraries can be made available for broader use. For example, at this time, Cas9-based forward screening for gain-of-function or knockout-mutants is based on the use of lentiviral gRNA libraries. While functional, this brings with it two significant downsides: i) Primarily, lentiviruses are strictly biosafety level 2 (BSL2) organisms due to their ability to integrate into the host genome, so their use is limited to laboratories certified for BSL2 work. Replication-deficient rAds are BSL1, as they can neither replicate nor insert their genome into the host. Using rAds, e.g., constructed according to embodiments of the present invention, as carrier for gRNA-libraries can significantly broaden the availability of this technology for research. ii) The second major advantage of rAd-based gRNA libraries, e.g., constructed according to embodiments of the present invention, is the ability, to co-express Cas9 nuclease via the same vector construct (e.g., a nucleic acid molecules of interest). Current lentiviral libraries do not carry a Cas9-gene, requiring either a stably expressing cell line or transient transfection to generate the desired effects. Moreover, with rAd, it is possible to reach a broader spectrum of cells, usually non-permissive to transfection or lentivirus transduction^{44,45}, but also establish Cas9-based mutagenesis within primary cells or even in vivo, which is not applicable with the currently available vector technologies.

Some exemplary embodiments of the invention are disclosed in the examples below. In embodiments the present method may comprise, e.g., as circular nucleic acid molecule, one or more of the plasmids pGPS1.1, p06-A5-GFP, pKD46, p06-fC5-GFP, p06-fC5-Lib2- GFP, p06-fC5-Lib3-GFP, p06-fC5-Lib4-GFP, pBWH-05-delE3, pBWH-fC5-E1Kn, pVfC5-Lib2, pVfC5-Lib3, pVfC5-Lib4, pAR-Int5-Cas9, pBWH-05-plX-Kan, pBWH-05-pIXZG, pBWH-05 and pKSB2.

In embodiments the present method may comprise, e.g., as circular nucleic acid molecule, one or more of the plasmids pO6-A5-mChe-WH, the warhead-modified species C BACs pBWH-C5-mChe, pBWH-C5-mChe-Cas9, pBWH-L-C5-mChe, pBWH-R-C5-mChe, and pBWH-C5-gRNA-mChe, species D BAC pBWH-D64M-GFP, species E BACs pBWH-E04 and pBHW-SE25, species B plasmid pLWH-B03, and species C plasmid pAC05-CE1.

In embodiments the present method may comprise, e.g., as circular nucleic acid molecule, one or more of the plasmids pAR-gRNA-Cas9-Amp, pAR-gRNA-Ex, pAR-gRNA-IntC5, pAR-gRNA-IntD64, pSG5-Cas9, pBAd5-mChe, pBAd5-FG40-GFP and/or pBWH-C5-mChe-DD-Cas9.

The contents of the inventor's previous application EP21778512.0 and WO2022/069523 and the methods described therein are enclosed herein by reference. The examples herein show the practical applicability of CTR (e.g., according to EP21778512.0 and WO2022/069523) in rescuing (reconstituting) the highly diverse libraries generated according to the present invention into viral vector libraries. The combination of the two methods developed by the present inventors expands in embodiments the scope of rAd-library applications from just highly diverse transgene carriers (e.g., gRNA libraries) to the generation of vector libraries for optimization of their performance.

Hence, in some embodiments the present means and methods may be combined with the means and methods described in EP21778512.0 and WO2022/069523, e.g., regarding methods for rescuing recombinant adenoviruses comprising a recombinant adenoviral genome.

The inventor's previous application EP21778512.0 and WO2022/069523 describe circular DNA molecules for rescuing recombinant adenoviruses comprising a recombinant adenoviral genome, preferably a human or simian adenoviral vector genome, with two inverted terminal repeats (ITRs) flanking the genome ends, wherein at least one of the ITRs is associated with a target sequence adjacent to a PAM sequence, wherein the target sequence is configured for generating an RNA-guided DNA endonuclease-mediated DNA double strand break at the external end of or in close proximity outside the external end of the respective ITR, preferably within less than about 15 nucleotides, a kit and methods for rescuing recombinant adenoviruses comprising or using a circular DNA molecule as described therein.

Hence, in some embodiments the invention also comprises a circular DNA molecule, e.g., a BAC, for rescuing recombinant adenoviruses comprising a recombinant adenoviral genome with two inverted terminal repeats (ITRs) flanking the genome ends, wherein
- at least one of the ITRs is associated with a target sequence adjacent to a PAM sequence, wherein
- the target sequence is configured for generating an RNA-guided DNA endonuclease-mediated DNA double strand break at the external end of or in *close proximity* outside the external end of the respective ITR, and

wherein optionally each of the two ITRs is associated with a target sequence adjacent to a PAM sequence, and/or
wherein optionally the target sequence or the PAM is located adjacent to the external end of the ITR, and/or
wherein optionally the molecule optionally comprises an expression cassette for at least one guide-RNA (gRNA) and/or an expression cassette for an RNA-guided DNA endonuclease generating DNA double strand breaks, such as S. pyogenes Cas9 (SpCas9), wherein the expression cassette(s) is/are located between the two ITRs outside the adenoviral genome.

In some embodiments the invention also comprises an in vitro method for rescuing recombinant adenoviruses, the method comprising
a. providing cells suited for rescuing recombinant adenoviruses, such as 293 cells,
b. introducing into said cell a circular DNA molecule according to the previous disclosure comprising a recombinant adenoviral genome,
   preferably with two inverted terminal repeats (ITRs) flanking the genome ends, wherein at least one of the ITRs is associated with a target sequence adjacent to a PAM sequence, wherein each of the target sequences is configured for generating an RNA-guided DNA endonuclease-mediated DNA double strand break at the external end of or in close proximity outside the external end of the respective ITR,
c. providing inside the cell an RNA-guided DNA endonuclease and at least one gRNA for targeting the RNA-guided DNA endonuclease to the target sequence of the circular DNA molecule,
d. linearizing recombinant adenoviral genome comprising the two ITRs inside the cells,
e. collecting viral particles from the cell supernatant.

Therefore, in one aspect the present invention relates to the use of recombinant virus(es) derived from the present nucleic acid molecule (that was generated according to embodiments of the present method) in the treatment or vaccination of a subject. In embodiments, recombinant viruses derived from the present nucleic acid molecule, e.g., derived or rescued by a method described herein or as described by the inventors before (e.g., in EP21778512.0 and WO2022/069523), may be used in the treatment or vaccination of a subject.

The person skilled in the art is familiar with methods and means for rescuing/deriving recombinant (adeno)viruses, e.g., from the present nucleic acid molecule (of interest), and any other suitable method may be used besides the ones referred to herein.

Each optional or preferred feature of the invention that is disclosed or described in the context of one aspect of the invention is herewith also disclosed in the context of the other aspects of the invention described herein. All features disclosed in the context of the method according to the invention described herein also relate to, and are herewith disclosed also in the context of, the nucleic acid molecule and the (therapeutic) uses, and vice versa.

### DETAILED DESCRIPTION OF THE INVENTION

All cited documents of the patent and non-patent literature are hereby incorporated by reference in their entirety.

The present invention is directed in one aspect to a method of constructing a nucleic acid molecule of interest, as well as a circular nucleic acid molecule constructed according to the method of the invention and its use in high-throughput screening approaches or the treatment of a genetic disease in a subject. The invention also relates to a pharmaceutical or immunogenic composition comprising the circular nucleic acid molecule according to the invention.

Herein a "subject" or "patient" may be a vertebrate, preferably a mammal, more preferably a human subject or patient. In the context of the present invention, the term "subject" or "patient" includes both humans and animals, particularly mammals, more particularly humans, and other organisms.

In the context of the present invention a therapeutic gene or therapeutic sequence may be a gene or nucleic acid sequence considered to cause or support a therapeutic effect in a subject and/or to prevent a disease or pathological genetic condition. In embodiments a therapeutic gene or therapeutic sequence may be a wildtype (wt) gene or nucleic acid (sequence) inserted into a subject possessing a mutated gene (e.g., which causes or negatively effects a disease), or, for example, the therapeutic gene or therapeutic sequence may be a gene or gene variant that induces a desired therapeutic effect in the subject.

One example of an *in vitro* fragment assembly technique is Gibson assembly, which is a sequence-independent and `seamless' cloning technique, e.g., employing PCR instead of restriction enzymes, thereby avoiding the existence of complete restriction enzyme target motifs in the generated nucleic acid molecule. In general, *in vitro* fragment assembly techniques often use short sequences at the 3' and 5' termini of sequence fragments that are intended to be assembled, wherein the short sequences are homologous or shared between certain of said sequence fragments, such that enzymes may assemble multiple fragment into one larger nucleic acid. Depending on the terminal sequences the order of assembly of the fragments may be predetermined.

Gibson assembly is a molecular cloning method that allows for the joining of multiple DNA fragments in a single, isothermal reaction. The entire Gibson assembly reaction requires few components and minor manipulations, such that the method enables the combination of up to 15 DNA fragments simultaneously based on sequence identity, wherein the DNA fragments are commonly required to comprise an overlap of 20-40 base pairs with neighboring DNA fragments. These DNA fragments are mixed with a cocktail of Exonuclease, DNA polymerase and DNA ligase and buffer components. For example, in a reaction or PCR reaction, the exonuclease digests the DNA from the 5' end, thereby not inhibiting the polymerase activity, such that reaction can proceed in a single process. The DNA polymerase incorporates nucleotides to close any gaps. The resulting single-stranded regions on adjacent DNA fragments are join together covalently by the DNA ligase eliminating any notches in the DNA, resulting in one joined DNA fragment. Either linear or closed circular molecules can be joined together. The method has the advantage that (compared to restriction enzyme-based approaches) no restriction site `scar' remains between two DNA fragments, as would be if restriction enzymes are used for the joining of nucleic acid fragments, e.g., PCR amplicons.

Endonucleases are enzymes that cleave the phosphodiester bond within a polynucleotide chain (e.g., of DNA or RNA). Some, endonucleases cleave DNA nonspecifically irrespectively of the nucleic acid sequence. Herein preferred endonuclease, commonly also referred to as restriction endonucleases or restriction enzymes, cleave only at specific nucleotide sequences. In general, restriction endonucleases or restriction enzymes cleave double-stranded DNA. A restriction endonuclease recognizes a specific DNA sequence (its target motif), and cleavage occurs usually within said recognition sequence (target motif) or in some distance from it, depending on the enzyme. Target motifs (recognition sequences) are commonly between 1-10, often between 4 and 8 base pairs (bp) long, and cleavage may result either in blunt ends or so called sticky ends (5' or 3' protruding ends). In general a sequence motif refers to a specific nucleic acid sequence, a sequence of consecutive nucleotides in a nucleic acid molecule.

In embodiments a fraction of a nucleic acid target motif (a partial target motif) of the (sequence specific) endonuclease comprises only a fraction/part of the minimal nucleic acid sequence motif required for the recognition and/or binding and/or cutting of the endonuclease to/of a nucleic acid molecule. Herein, a 'half site' refers to a nucleic acid sequence that represents a partial restriction endonuclease (sequence specific endonuclease) target motif (the nucleic acid sequence at which the endonuclease binds and/or cuts the nucleic acid). Accordingly, a 'half site' refers to a fraction of the nucleic acid sequence of the endonuclease target motif.

Herein a 'complete' target motif of an (sequence specific) endonuclease or restriction enzyme refers to a nucleic acid sequence motif that enables the recognition, binding and/or cutting (digestion) of a nucleic acid strand by an endonuclease or restriction enzyme. 'Complete' refers to a nucleic acid sequence, preferably about 100% of the minimum (shortest) required sequence, which enables the desired effect achieved by the respective endonuclease/restriction enzyme, which is preferably the binding and/or digestion/cutting of the nucleic acid strand by said endonuclease/restriction enzyme. In other words, the 'complete' target nucleic acid motif preferably comprises at least the minimum/minimal target nucleic acid sequence (consecutive sequence of nucleotides) of the respective endonuclease or restriction enzyme that is required for a recognition and/or cutting by said enzyme. The skilled person knows how to determine and design a 'complete' target motif of an endonuclease or restriction enzyme (e.g., cutting site). In embodiments to create complete endonuclease target motifs on the basis of partial target motifs (`half sites'; nucleic acid motifs that comprise only a fraction of the nucleic acid sequence of the complete target motif for the endonuclease) it is preferred that one forward and one reverse sequence motif is selected. In embodiments two complete target sequence motifs are generated, preferably one at the 5' of the cloning site of interest (the intended site of mutagenesis) and the other at the 3' of the cloning site of interest.

In the context of the present invention the term of a 'cloning site' or 'cloning site of interest' preferably broadly refers to a nucleic acid sequence position in a (circular) nucleic acid molecule at which another nucleic acid sequence (fragment or molecule) should be inserted and/or be removed/deleted from. Herein, the meaning of the term 'cloning site (of interest)' preferably extends beyond its strict meaning used sometimes in molecular biology referring to a site of genetic insertion of a 'foreign' or synthetic nucleic acid sequence. In embodiments, this may be one application of the present method, however, in some embodiments the present method may also comprise or be used for the insertion of a modification (mutation) of the sequence of the (circular) nucleic acid molecule (itself), e.g., of an (adeno)viral nucleic acid sequence therein, in addition or instead of the insertion of a certain 'foreign' sequence of interest. Hence, in some of said embodiments, the nucleic acid sequence of interest inserted in step f. into the cloning site of interest may comprise a sequence comprising one or more mutations, e.g., of a (adeno)viral sequence.

In embodiments the fraction of the nucleic acid target motif (partial target motif) of the (sequence specific) endonuclease comprises between 1-99 %, between 5-95 %, between 10-90 %, between 20-80 %, between 30-70 % between 40-60% or preferably essentially half, e.g., between 40-60 %, or about 1%, 2.5%, 5%, 10 %, 15%, 20%, 25%, 30%, 35%, 40%, 45%, 50%, 55%, 65%, 70%, 75%, 80%, 85%, 90%, 95, or even 99% of the nucleic acid sequence of the complete target motif for the endonuclease. In embodiments `essentially half' may in embodiments refer to a fraction, or to a fraction of about 50%, of between 40-60%, or even of between 30-70%. Essentially half of a nucleic acid sequence motif may refer in embodiments to a nucleic acid sequence comprising between 30-70%, or between 40-60%, or of about 50% of a sequence motif referred to (e.g., a 'complete' target sequence motif of an endonuclease).

Commonly an (antibiotic) resistance gene contains information for the production of a protein that renders an (antibiotic) substance ineffective and hence confers resistance against an (antibiotic) substance to a microorganism comprising said resistance gene.

Microorganisms are in the context of the invention preferably bacteria, or yeasts. Alternatively to a microorganism here also a eukaryotic, or even mammalian or insect, cell or cell line may be used for selecting and/or amplifying the nucleic acid molecule at any step of the present method. Microorganisms that may be used for the selection and/or amplification of nucleic acids, such as circular nucleic acids and/or vectors, are preferably bacteria or yeasts. The skilled person is aware how to select and use microorganisms for the selection, replication and/or amplification of nucleic acids. During the amplification a selection process may also occur in a microorganism, e.g., if a nucleic acid molecule of interest comprises an antibiotic, e.g., antibacterial and/or antifungal, resistance gene. Thereby the microorganism may be cultivated in the presence of said antibiotic, such that only microorganisms comprising the nucleic acid molecule of interest are propagated/selectively amplified and the nucleic acid molecule of interest is selected and/or amplified thereby. This process commonly involves the transfer of a nucleic acid of interest into the microorganism with suitable means (known to the skilled person), and its subsequent isolation/recovery from the microorganism after the selection and/or amplification with suitable means (known to the skilled person).

Homologous recombination is one kind of genetic recombination in which nucleotide sequences are exchanged between two n double-stranded or single-stranded nucleic acids (usually DNA as in cellular organisms but may be also RNA in viruses) comprising (long) stretches of similar or identical sequence. For example, double-strand breaks in DNA can be repaired through homologous recombination. In embodiments homologous recombination may occur between two (linear) DNA sequences containing identical or similar sequences of between 4-100 homologous bases, such that genetic material is exchanged between the two strands of DNA. In embodiments herein the partial target motifs of endonucleases in the circular nucleic acid molecule may be flanked by similar, identical or homologous nucleic acid sequences, such that homologous recombination can occur in the context of the present method, as described herein.

In the context of the invention an "immunogenic composition" may refer to a foreign substance, e.g., an antigen, to induce an immune response in the body of a subject, e.g., a human or other animal. Herein immunogenicity described the ability to induce a humoral and/or cell-mediated immune response.

In the context of the invention a "vaccine" refers to a biological formulation providing active acquired immunity to a particular infectious condition. A vaccine commonly comprises an agent that resembles a disease-causing pathogen and is often made from weakened or killed forms of the microorganism, its toxins, or one of its surface proteins, or with nucleic acid molecules encoding a relevant antigen. The vaccine stimulates a subject's immune system to recognize it as a threat, eliminate it, and to further recognize and eliminate any of the pathogens associated with that vaccine that it may encounter in the future.

As used herein, the terms "comprising" and "including" or grammatical variants thereof are to be taken as specifying the stated features, integers, steps or components but do not preclude the addition of one or more additional features, integers, steps, components or groups thereof. This term encompasses the terms "consisting of' and "consisting essentially of. Thus, the terms "comprising"/"including"/"having" mean that any further component (or likewise features, integers, steps and the like) can/may be present. The term "consisting of means that no further component (or likewise features, integers, steps and the like) is present.

Adenoviruses are members of the family Adenoviridae and have a medium size of about 90-100 nm. They are nonenveloped (without an outer lipid bilayer) viruses with an icosahedral nucleocapsid containing a double stranded DNA genome. They have a broad range of vertebrate hosts; in humans, more than 50 distinct adenoviral serotypes have been found to cause a wide range of illnesses, from mild respiratory infections in young children (known as the common cold) to life-threatening multi-organ disease in people with a weakened immune system. The present invention is directed to provision of DNA molecules as well as methods and material for rescuing recombinantn adenoviruses of all kinds. However, human and simian adenoviruses are preferred. Furthermore, the invention also relates to rescue of adenoviral vectors, which are derived from all kinds of adenoviruses, such as human or simian adenoviruses of various serotypes.

Classification of Adenoviridae can be complex. In humans, there are about 100 accepted human adenovirus types in seven species (Human adenovirus A to G; according to the ICTV 9th Report (2011) available under: https://talk.ictvonline.org/ictv-reports/ictv 9th_report/dsdna-viruses-2011/w/dsdna_viruses/93/adenoviridae; see also Lefkowitz et al. Nucleic Acids Res. 2018 Jan 4;46(D1):D708-D717, doi: 10.1093/nar/gkx932). The numbering of the types (between 1-57) are identical with the earlier used numbers for the serotypes: A: 12, 18, 31; B: 3, 7, 11, 14, 16, 21, 34, 35, 50, 55; C: 1, 2, 5, 6, 57; D: 8, 9, 10, 13, 15, 17, 19, 20, 22, 23, 24, 25, 26, 27, 28, 29, 30, 32, 33, 36, 37, 38, 39, 42, 43, 44, 45, 46, 47, 48, 49, 51, 53, 54, 56; E: 4; F: 40, 41; G: 52. For isolates, which could not be classified by serology and the newly identified types a number was/is given by the ICTV committee in the order of their acceptance. The present invention comprises and is useful for all know adenovirus types, and it can be expected that it is also applicable to newly identified types of adenoviruses, which carry terminal ITR.

Different types/serotypes are associated with different conditions: respiratory disease is mainly species HAdV-B and C; conjunctivitis often occurs with HAdV-B and D; gastroenteritis is often associated with HAdV-F types 40, 41, HAdV-G type 52; obesity or adipogenesis are often induced by HAdV-A type 31, HAdV-C type 5, HAdV-D types 9, 36, 37.

The present invention is not restricted to adenoviruses infecting humans (herein referred to as human adenovirus or (if referring to the viral genome) human adenoviral genome), but also comprises adenoviruses infecting other animals, such as monkey or chimpanzees.

Comprised are in particular simian mastadenoviruses, in particular simian mastadenovirus A, B and E, are adenoviruses infecting simians. Also comprised are adenoviruses that have initially been isolated from chimpanzees, which may be classified into "human" adenovirus species because of their great similarity to certain human adenoviruses (HAdVs). For example, the simian adenoviruses SAdV-22 to SAdV-25 belong to the species human mastadenovirus E and SAdV-21 to the species human mastadenovirus B.

When not restricting the subject to human viruses, Adenoviridae can be divided into five genera: Mastadenovirus, Aviadenovirus, Atadenovirus, Siadenovirus, and Ichtadenovirus, all of which can be subject of the present invention.

Adenoviruses represent the largest known nonenveloped viruses which can be transported through the endosome (i.e., envelope fusion is not necessary). The virion also has a unique "spike" or fiber associated with each penton base of the capsid that aids in attachment to the host cell via the receptor on the surface of the host cell.

The adenovirus genome comprised by the virion (viral particle) is linear, non-segmented double-stranded (ds) DNA that is usually between 26 and 48 Kbp. This allows the virus to theoretically carry 22 to 40 genes. The viral genome has a terminal 55 kDa protein associated with each of the 5' ends of the linear dsDNA. These are used as "primers" in viral replication and ensure that the ends of the virus' linear genome are adequately replicated. rAd genomes can be constructed in E. *coli* where their genomes can be maintained, propagated and modified in a form of circular plasmid or bacterial artificial chromosome (BACs).

In embodiments the circular DNA molecule of the invention, e.g., comprising a recombinant adenoviral genome as nucleic acid of interest, the DNA sequence of e.g., the recombinant adenoviral genome to be packaged into a viral particle, is preferably comprised by the circular DNA molecule. In embodiments the circular DNA molecule comprising the sequence of the recombinant adenoviral genome is introduced into a producing cell, e.g., a microorganism or other eucaryotic cell. In embodiments it may be linearized therein as described herein or by means of an RNA-guided endonuclease, preferably using the CRISPR/Cas system, and the resulting linear DNA molecule comprising or consisting of the sequence of the recombinant adenoviral genome can be replicated and packaged into viral particles produces by the cells.

Adenoviruses possess a linear dsDNA genome which is replicated in the nucleus of vertebrate cells using the host's replication machinery. Entry into the host cell is initiated by the knob domain of the fiber protein binding to the cell receptor, such as CD46 for the group B human adenovirus serotypes and the coxsackievirus adenovirus receptor (CAR) for all other serotypes. There are some reports suggesting MHC molecules and sialic acid residues functioning in this capacity as well. This first interaction is followed by a secondary interaction, where a motif in the penton base protein (see capsomere) interacts with αv integrin, functioning as a co-receptor interaction stimulating entry of the adenovirus. Binding to αv integrin results in endocytosis of the virus particle via clathrin-coated pits. Attachment to αv integrin stimulates cell signaling and thus induces actin polymerization resulting in entry of the virion into the host cell within an endosome.

Once the virus has entered the host cell, the endosome acidifies, which alters virus topology by causing capsid components to disband. The capsid is destabilized, and protein VI is released from the capsid. These changes, as well as the toxic nature of the pentons, destroy the endosome, resulting in the movement of the virion into the cytoplasm. With the help of cellular microtubules, the virus is transported to the nuclear pore complex, whereby the adenovirus particle disassembles. Viral DNA is then released and can enter the nucleus. After this the DNA associates with histone molecules and viral gene expression can occur and new virus particles can be generated. The adenovirus life cycle is divided in two phases: an early and a late phase. In both phases, a primary transcript that is alternatively spliced to generate monocistronic mRNAs compatible with the host's ribosome is generated, allowing for the products to be translated. The early genes are responsible for expressing mainly non-structural, regulatory proteins. The goal of these proteins is threefold: to alter the expression of host proteins that are necessary for DNA synthesis; to activate other virus genes (such as the virus-encoded DNA polymerase); and to avoid premature death of the infected cell by the host-immune defenses (blockage of apoptosis, blockage of interferon activity, and blockage of MHC class I translocation and expression). DNA replication separates the early and late phases. Once the early genes have liberated adequate virus proteins, replication machinery, and replication substrates, replication of the adenovirus genome can occur. A terminal protein that is covalently bound to the 5' end of the adenovirus genome acts as a primer for replication. The viral DNA polymerase then uses a strand displacement mechanism, as opposed to the conventional Okazaki fragments used in mammalian DNA replication, to replicate the genome. The late phase of the adenovirus lifecycle is focused on producing sufficient quantities of structural protein to pack all the genetic material produced by DNA replication. Once the viral components have successfully been replicated, the virus is assembled into its protein shells and released from the cell as a result of virally induced cell lysis.

Adenovirus (Ad) has received tremendous attention as an effective gene delivery vector and was in fact the first DNA virus to enter rigorous therapeutic development, due to its well-defined biology, its genetic stability, its high gene transduction efficiency and its ease of large-scale production. Ad serotypes differ in tropism and are further divided into six subgroups, A-G. Differences in viral capsids delineate tropisms among serotypes. The viral capsid is comprised of capsid proteins, core proteins, and cement proteins. These diverse serotypes can give rise to a vast range of therapeutic candidate viruses.

Adenoviral vectors offer important advantages: First, adenovirus is the most effective means of delivering genes in vivo as most human cells express the primary adenovirus receptor and the secondary integrin receptors. Thus, are easily infected with adenovirus vectors and consequently yield high levels of the transgene expression. Second, the development of "gutless" adenoviral vectors allows us to circumvent anti-adenoviral vector immunity. Third, there has been extensive experience with adenovirus vectors in many different clinical applications, and the safest dosing and routes of administration are well established. Fourth, adenovirus vectors offer a versatile platform for developing strategies to modify viral capsids in order to enhance therapeutic properties and improve targeting specificity of the virus. Interestingly, some of the inherited shortcomings of adenovirus, such as immunity evoked against the adenovirus capsid and low-level expression of adenovirus genes, may now prove beneficial for the development of anticancer immunotherapies, where inducing immunity against the cancer or directly killing the cancer cell is the goal. Furthermore, the combined immunity against the adenovirus together with the short time of expression is ideal for using the adenovirus as a platform for developing vaccines. However, depending on the application, the use of the rare serotypes 2 and 5 to construct adenoviral vectors for gene therapy can be advantageous to avoid preexisting immunity. Adenoviral vectors can transduce both replicating and quiescent cell populations, making them a valuable tool in delivering transgenes in vivo and within mature tissues. In contrast to lentiviral vectors, adenoviral vectors can deliver larger transgenes up to 8 kbp in size; however, their DNA does not integrate into the host genome, but rather, resides episomally in the host nucleus. Such episomal transduction precludes the risks of insertional mutagenesis, without direct integration into the host genome.

The adenoviral genomes are linear, non-segmented double-stranded DNA with sizes ranging from 26 kb to 45 kb in length, depending on the serotype. The genome of the commonly used human adenovirus type 5 (HAdV-5) is approximately 36 kb. The genomic DNA or genomic sequence is flanked on both ends by hair-pin-like, inverted terminal repeats (ITR), which serve a variety of purposes. One of the roles of ITRs is to act as a self-primer to promote primase-independent DNA synthesis, making them important elements in DNA multiplication. Another function of the ITRs is to facilitate integration into the host genome. In addition to ITRs, another genetic element of the adenovirus is the packaging signal, which is located on the left arm of the genome and is required for proper viral transcript packaging. Viral transcripts are classified as either early or late. The four early transcriptional units, E1, E2, E3, and E4, are responsible for expressing non-structural proteins having regulatory functions in particular in viral DNA replication. The late proteins encode for structural components of the Ad virion.

To establish safety in use of adenoviral vectors, essential viral-replication genes can be eliminated. First generation adenoviral vectors are those stripped of regulatory genes E1a and E1b - the first transcriptional regulatory factors to be produced during the viral life cycle. The depletion of this gene resulted in replication-deficient adenoviral vectors with an initial transgene cloning capacity of 5.2 kb. To increase cloning capacity and continue to decrease capacity for viral replication, second generation adenoviral vectors were developed by deletion of other non-structural genes (E2/E3/E4) in addition to the original E1 gene absent in first generation vectors. While the second generation of vectors demonstrated increased cloning capacity and reduced cytotoxicity, they still triggered immune responses in vivo resulting in the reduced yields of transduced cells. To develop less immunogenic vectors, third generation of adenoviral vectors were developed. These are also termed high-capacity adenoviral vectors (HC-AdVs), also known as gutless AdVs or helper-dependent AdVs (HD-AdVs). The HC-AdV is stripped of all viral coding sequences, resulting in a vector with only 5' and 3' ITRs in addition to a packaging signal, thus providing a larger capacity for transgenic cloning sequences (36 kb). Furthermore, the structure of the HC-AdV minimizes cytotoxicity, thus enabling prolonged expression of therapeutic genes, rendering the HC-AdV the most promising AdV to use for gene therapy to date. HC-AdVs are beneficial because they lack the viral elements that can cause an immune response in the host. HC-AdV's are deemed "helper-dependent" because while these vectors lack viral genetic components, they also lack the necessary packaging components. A complementary virus, or helper virus (HV), can be used to provide the necessary proteins in trans for the packaging of an Ad-based vector. The HV is not packaged along with the desired HC-AdV because it has its packaging sequences flanked by IoxP recognition sites, which is sufficiently excised by Cre recombinase so that the helper virus DNA remains unpackaged. While the packaging ability of the HV is stunted, the HV is replicated at normal levels and can thus express all of the functions necessary in trans for replication and packaging of a vector genome containing the appropriate cis-acting elements.

The generation of recombinant adenoviral vectors and the generation of the required recombinant adenoviral genomes has been described in detail by Lee et al (Genes Dis. 2017 Jun; 4(2): 43-63. doi: 10.1016/j.gendis.2017.04.001) and the required techniques are known to the person skilled in the art. Generation of such adenoviral genomes is also evident from the description of the examples below.

At least four commonly used methods to generate and produce adenovirus vectors for gene delivery have been developed, which all require linearization of the recombinant adenoviral genome. The first, traditional method uses recombination in HEK-293 cells. The gene of interest (GOI) is first cloned into a shuttle vector, which contains 5'-ITR, packaging signal and homologous regions to adenoviral genome. Adenoviruses are generated in HEK-293 cells through recombination between shuttle vector, which has to be linearized prior to transfection into the producing cells, and adenoviral backbone vector, which is unable to produce virus by itself. A second approach uses Cre/LoxP-mediated recombination. The GOI is cloned into a shuttle vector that contains LoxP site(s). Cre recombinase-mediated recombination occurs with a LoxP-containing adenoviral backbone vector in vitro or 293-Cre cells, leading to the generation of adenoviruses. A third approach uses the AdEasy system. The GOI is subcloned into a shuttle vector that contains 5'-ITR and packaging signal, as well as a kanamycin-containing bacterial replication unit flanked with homologous arms. Recombinant adenoviral plasmids are generated through homologous recombination between the linearized shuttle vector and ampicillin-resistant adenoviral backbone vector, such as pAdEasy1, in the bacterial strain BJ5183 cells under kanamycin selection. The resultant adenoviral plasmids are linearized and used for adenovirus production in HEK-293 cells. A fourth approach uses helper adenovirus for the production of HC-AdVs (or HD-AdVs, or Gutless AdVs). The GOI is cloned into a transfer vector that contains both ITRs and packaging signal only. Adenoviruses are generated with a helper adenovirus, which will not be packaged due to the deletion of packaging signal in the modified HEK-293 cells, usually through Cre/LoxP or FLP/FRT excision system.

The term "recombinant" adenoviral genome refers to the fact that the genomic DNA sequences of the adenoviral genome comprised by the circular nucleic acid molecule of the invention is formed or assembled by laboratory methods of genetic recombination, such as molecular cloning, to bring together genetic material from multiple sources, creating sequences that would not otherwise be found. Additionally, in the context of adenoviral genomes it is understood that the term also refers to viral genomes resulting from recombination between virus genomes in a cell infected by more than one virus strain, which can occur by homologous recombination of the nucleic acid strands of the genomes of the different virus strains.

A circular nucleic acid , e.g., DNA, molecule of the invention comprising a recombinant adenoviral genome can be any suitable kind of circular DNA molecule, such as preferably a bacterial artificial chromosome (BAC) or a plasmid, preferably a high copy plasmid.

Adenoviral vectors can have large genomes (i.e., 36kb) making genetic manipulations by classical cloning strategies difficult and ineffective. However, there are virus cloning technologies using BACs that benefit from the large cloning capacity of BACs. A bacterial artificial chromosome (BAC) is a DNA construct, based on a functional fertility plasmid (or F-plasmid), used for transforming and cloning in bacteria, usually *E. coli.* F-plasmids play a crucial role because they contain partition genes that promote the even distribution of plasmids after bacterial cell division. The bacterial artificial chromosome's usual insert size is 150-350 kbp. A similar cloning vector called a PAC has also been produced from the DNA of P1 bacteriophage and can also be used in embodiments of the invention.

Plasmids are circular DNA molecules. Inside a cell, plasmids are physically separated from chromosomal DNA and can replicate independently. They are most commonly found as small circular, double-stranded DNA molecules in bacteria; however, plasmids are sometimes present in archaea and eukaryotic organisms. In nature, plasmids often carry genes that benefit the survival of the organism and confer selective advantage such as antibiotic resistance. While chromosomes are large and contain all the essential genetic information for living under normal conditions, plasmids are usually comparably small and contain only additional genes that may be useful in certain situations or conditions.

Adenoviruses are non-enveloped viruses (without an outer lipid bilayer) that comprise a capsid, majorly composed of two coat proteins (hexon and penton) and the associated protein fiber. Recombinant adenovirus (rAd) and recombinant adeno-associated virus (rAAV) are frequently extensively used vehicles for gene transfer applications, such as gene therapy *in vitro* and *in vivo.*

Herein PCR fragment preferably refers to a nucleic acid molecule generated by Polymerase Chain Reaction (PCR).

gRNA commonly refers in the context of CRISPR-Cas systems to a guide RNA (gRNA), which is a specific RNA sequence that recognizes a nucleic acid sequence region of interest in a target nucleic acid sequence and subsequently binds the Cas9 protein thereby directing it to the target nucleic acid sequence (target sequence motif) to perform a modification process. Therefore, an RNA-guided DNA endonuclease can be provided together with at least one gRNA for targeting the RNA-guided DNA endonuclease to the target sequence in a DNA molecule. In embodiments a nucleic acid sequence of interest may encode an RNA-guided DNA endonuclease and/or at least one (corresponding) gRNA.

CRISPR is an abbreviation of Clustered Regularly Interspaced Short Palindromic Repeats and is a family of DNA sequences in bacteria. The sequences contain snippets of DNA from viruses that have attacked the bacterium. These snippets are used by the bacterium to detect and destroy DNA from further attacks by similar viruses. These sequences play a key role in a bacterial defense system and form the basis of a technology known as CRISPR/Cas that effectively and specifically changes genes within organisms.

Sequences of the CRISPR loci are transcribed and processed into CRISPR RNAs (crRNAs) which, together with a trans-activating crRNAs (tracrRNAs), complex with CRISPR-associated (Cas) proteins to dictate specificity of DNA cleavage by Cas nucleases through Watson-Crick base pairing between nucleic acids (Wiedenheft, B et al (2012). Nature 482: 331-338; Horvath, P et al (2010). Science 327: 167-170; Fineran, PC et a. (2012). Virology 434: 202-209). It was shown that the three components required for the type II CRISPR nuclease system are the Cas9 protein, the mature crRNA and the tracrRNA, which can be reduced to two components by fusion of the crRNA and tracrRNA into a single guide RNA (sgRNA) and that re-targeting of the Cas9/sgRNA complex to new sites could be accomplished by altering the sequence of a short portion of the gRNA (Garneau, JE et al (2010). Nature 468: 67-71; Deltcheva, E et al. (2011). Nature 471: 602-607, Jinek, M et al (2012) Science 337: 816-821). CRISPR-Cas systems are RNA-guided adaptive immune systems of bacteria.

It was demonstrated that the CRISPR/Cas9 system could be engineered for modification of double stranded DNA molecules inside a cell, for example efficient genetic in mammalian cells. The only sequence limitation of the CRISPR/Cas system appears to derive from the necessity of a protospacer-adjacent motif (PAM) located immediately 3' to the target sequence. The PAM sequence is specific to the species of Cas9. For example, the PAM sequence 5'-NGG-3' is necessary for binding and cleavage of DNA by the commonly used Cas9 from Streptococcus pyogenes. However, Cas9 variants with novel PAMs have been and may be engineered by directed evolution, thus dramatically expanding the number of potential target sequences. Cas9 complexed with the crRNA and tracrRNA undergoes a conformational change and associates with PAM motifs throughout the genome interrogating the sequence directly upstream to determine sequence complementarity with the gRNA. The formation of a DNA-RNA heteroduplex at a matched target sequence motif allows for cleavage of the target DNA by the Cas9-RNA complex. These methods and mechanisms are well known in the art.

As known in the art, CRISPR/Cas9 has been exploited to develop potent tools for genome manipulation in animals, plants and microorganisms, but, can also be exploited for manipulation of exogenous DNA molecules that have been introduced into a host cell. The RNA-guided Cas9 endonuclease first recognizes a 2- to 4-base-pair conserved sequence named the protospacer-adjacent motif (PAM), which flanks a target DNA site. Upon binding to the PAM, Cas9 interrogates the flanking DNA sequences for base-pairing complementarity to a guide RNA. If there is complementarity between the first 12 base pairs (the `seed' sequence) of the guide RNA and the target DNA strand, RNA strand invasion accompanies local DNA unwinding to form an R-loop. Precise cleavage of each DNA strand by the RuvC and HNH domains of Cas9 generates a blunt double-strand DNA (dsDNA) break (DSB) at a position three base pairs upstream of the 3' edge of the protospacer sequence, measuring from the PAM.'This DSB inducing activity of Cas9 as a preferred RNA-guided DNA endonuclease is exploited by the present invention for generating DSB in the circular DNA molecule of the invention after introduction into a producing cell comprising suitable gRNA and Cas9.

CRISPR/Cas9 genome-editing experiments have been exploiting the host cell machinery to repair the genome precisely at the site of the Cas9-generated DSB. Mutations can arise either by non-homologous end joining (NHEJ) or homology-directed repair (HDR) of DSBs. NHEJ can produce small insertions or deletions (INDELs) at the cleavage site, whereas HDR uses a native (or engineered) DNA template to replace the targeted allele with an alternative sequence by recombination. Additional DNA repair pathways such as single-strand annealing, alternative end joining, microhomology-mediated joining, mismatch and base- and nucleotide-excision repair can also produce genome edits.

Cas9 variants derived from the Streptococcus pyogenes Cas9 (SpCas9) have been generated for use as nickases, dual nickases or Fokl fusion variants. More recently, Cas9 orthologs, and other nucleases derived from class 2 CRISPR-Cas systems including Cpf1 and C2c1, have been added to the CRISPR toolbox. These ongoing efforts to mine the abundant bacterial and archaeal CRISPR-Cas systems should increase the range of molecular tools available to researchers.

In the context of the present invention, the term "RNA-guided DNA endonuclease" refers to DNA endonucleases that interact with at least one RNA-Molecule. In the context of the present invention the terms RNA-guided DNA endonuclease and RNA-guided endonuclease are used interchangeably. DNA endonucleases are enzymes that cleave the phosphodiester bond within a DNA polynucleotide chain. In case of RNA-guided DNA endonuclease, the interacting RNA-molecule may guide the RNA-guided DNA endonuclease to the site or location in a DNA where the endonuclease becomes active. In particular, the term RNA-guided DNA endonuclease refers to naturally occurring or genetically modified Cas nuclease components or CRISPR-Cas systems, which include, without limitation, multi-subunit Cas protein effectors of class 1 CRISPR-Cas systems as well as single large effector Cas proteins of class 2 systems. Details of the technical application of CRISPR/Cas systems and suitable RNA-guided endonuclease are known to the skilled person and have been described in detail in the literature, as for example by Barrangou R et al. (Nat Biotechnol. 2016 Sep 8;34(9):933-941), Maeder ML et al. (Mol Ther. 2016 Mar;24(3):430-46) and Cebrian-Serrano A et al. (Mamm Genome. 2017; 28(7): 247-261). The present invention is not limited to the use specific RNA-guided endonucleases and therefore comprises the use of any given RNA-guided endonucleases in the sense of the present invention suitable for use in the method described herein.

Any RNA-guided DNA endonuclease known in the art may be employed in accordance with the present invention. RNA-guided DNA endonuclease comprise, without limitation, Cas proteins of class 1 CRISPR-Cas systems, such as Cas3, Cas8a, Cas5, Cas8b, Cas8c, Cas10d, Cse1, Cse2, Csy1, Csy2, Csy3, GSU0054, Cas10, Csm2, Cmr5, Csx11, Csx10 and Csf1; Cas proteins of class 2 CRISPR-Cas systems, such as Cas9, Csn2, Cas4, Cpf1, C2c1, C2c3 and C2c2; corresponding orthologous enzymes/CRISPR effectors from various bacterial and archeal species; engineered CRISPR effectors with for example novel PAM specificities, increased fidelity, such as SpCas9-HF1/eSpCas9, or altered functions, such as nickases. Particularly preferred RNA-guided DNA endonuclease of the present invention are Streptococcus pyogenes Cas9 (SpCas9), Staphylococcus aureus Cas9, Streptococcus thermophilus Cas9, Neisseria meningitidis Cas9 (NmCas9), Francisella novicida Cas9 (FnCas9), Campylobacter jejuni Cas9 (CjCas9), Cas12a (Cpf1) and Cas13a (C2C2) (Makarova KS et al. (November 2015). Nature Reviews Microbiology. 13 (11): 722-36).

The person skilled in the art is aware of how to use alternative Crispr/Cas systems as well as tools and methods that provide or allow the gain of information on the details of such alternative systems. In embodiments, the DNA molecule of the invention can be configured for generation of DSB mediated by a different RNA-guided endonuclease than Cas9 or SpCas9, such as for example Cpf1. Cpf1 requires one associated guide RNA for generating staggered/sticky end cuts and it cuts in non-dividing cells, such as nerve cells. The RNA-guided DNA endonuclease and in particular Cas9 may also be a modified protein, wherein the nuclease function of the protein is altered into a nicking endonuclease function, which only cuts one of the two DNA strands of the dsDNA. In other words, the naturally occurring endonucleases function of cleaving both strands of a double-stranded target DNA, is altered into an endonuclease that cleaves (i.e., nicks) only one of the strands. Such modified RNA-guided DNA endonucleases are also called "nickases" in the context of the present invention. Means and methods of modifying RNA-guided DNA endonuclease such as Cas9 accordingly are well known in the art and include for example the introduction of amino acid replacements into Cas9 that render one of the nuclease domains inactive. More specifically, aspartate can be replaced against alanine at position 10 of the Streptococcus pyogenes Cas9 (SpCas9 D10A; Cong et al. (2013) Science 339:819-823). Further examples are known in the art, for example the H840A replacement in SpCas9 (Mali P et al. Nat Biotechnol. 2013 Sep; 31(9):833-8; Ran FA et al. Cell. 2013 Sep 12; 154(6): 1380-9).

In embodiments of the method of the invention, the RNA-guided DNA endonuclease may be introduced as a protein, but alternatively the RNA-guided DNA endonuclease may also be introduced in form of a nucleic acid (of interest) encoding said protein. It will be appreciated that the nucleic acid molecule encodes said RNA-guided DNA endonuclease in expressible form such that expression in the cell results in a functional RNA-guided DNA endonuclease protein such as Cas9 protein. Means and methods to ensure expression of a functional polypeptide are well known in the art. For example, the coding sequences for the endonuclease may be comprised in a vector, such as for example a plasmid, cosmid, virus, bacteriophage or another vector used conventionally e.g., in genetic engineering. The coding sequences inserted in the vector can e.g., be synthesized by standard methods or isolated from natural sources. The coding sequences may further be ligated to transcriptional regulatory elements and/or to other amino acid encoding sequences. Such regulatory sequences are well known to those skilled in the art and include, without being limiting, regulatory sequences ensuring the initiation of transcription, internal ribosomal entry sites (IRES) and optionally regulatory elements ensuring termination of transcription and stabilization of the transcript. Non-limiting examples for regulatory elements ensuring the initiation of transcription comprise a translation initiation codon, transcriptional enhancers such as e.g. the SV40-enhancer, insulators and/or promoters, such as for example the cytomegalovirus (CMV) promoter, SV40-promoter, RSV-promoter (Rous sarcome virus), the lacZ promoter, chicken beta-actin promoter, CAG-promoter (a combination of chicken beta-actin promoter and cytomegalovirus immediate-early enhancer), the gai10 promoter, human elongation factor 1a-promoter, A0X1 promoter, GAL1 promoter CaM-kinase promoter, the lac, trp or tac promoter, the lacUV5 promoter, the autographa californica multiple nuclear polyhedrosis virus (AcMNPV) polyhedral promoter or a globin intron in mammalian and other animal cells. Non-limiting examples for regulatory elements ensuring transcription termination include the V40-poly-A site, the tk-poly-A site or the SV40, lacZ or AcMNPV polyhedral polyadenylation signals, which are to be included downstream of the nucleic acid sequence of the invention. Additional regulatory elements may include translational enhancers, Kozak sequences and intervening sequences flanked by donor and acceptor sites for RNA splicing. Moreover, elements such as origin of replication, drug resistance gene or regulators (as part of an inducible promoter) may also be included. Nucleic acid molecules encoding said RNA-guided DNA endonuclease include DNA, such as cDNA or genomic DNA, as well as RNA and in particular mRNA. It will be readily appreciated by the skilled person that more than one nucleic acid molecule may encode an RNA-guided DNA endonuclease in accordance with the present invention due to the degeneracy of the genetic code. Degeneracy results because a triplet code designates 20 amino acids and a stop codon. Because four bases exist which are utilized to encode genetic information, triplet codons are required to produce at least 21 different codes. The possible e possibilities for bases in triplets give 64 possible codons, meaning that some degeneracy must exist. As result, some amino acids are encoded by more than one triplet, i.e., by up to six. The degeneracy mostly arises from alterations in the third position in a triplet. This means that nucleic acid molecules having different sequences, but still encoding the same RNA-guided DNA endonuclease, can be employed in accordance with the present invention.

In embodiments of the present invention the sequence of interest may comprise a nucleic acid sequence encoding a Cas9 nuclease and/or cDNA encoding gRNA and/or any other protein or nucleic acid sequence described herein and useful for the application/functioning of the Crispr/Cas system, e.g., for genetic manipulation of a target nucleic acid, a subject, cell or cell line.

In embodiment the nucleic acid molecules used in accordance with the present invention may be of natural as well as of (semi) synthetic origin. Thus, the nucleic acid molecules may, for example, be nucleic acid molecules that have been synthesized according to conventional protocols of organic chemistry. The person skilled in the art is familiar with the preparation and the use of said probes (see, e.g., Sambrook and Russel "Molecular Cloning, A Laboratory Manual", Cold Spring Harbor Laboratory, N.Y. (2001)).

In preferred embodiments, the RNA-guided endonuclease generates blunt ends. In alternative embodiments, the RNA-guided endonuclease generates sticky ends. In the context of the present invention, double strand breaks can occur due to cleavage of both strands by one RNA-guided endonuclease or due to two single-strand cuts on both the (+)- and the (-)-strand by nickases.

A double strand break can be generated by cleavage of both strands of the dsDNA at the same/corresponding position on the complementary strands, leading to the formation of blunt ends of the resulting separated ends of the dsDNA molecule, as it is mostly the case for Cas9 mediated cleavage. However, in case of non-canonical cleavage, Cas9 may also induce the formation of double strand breaks with sticky/staggered ends, wherein the strand breaks on the two complementary DNA strands of the dsDNA are located at different positions, leading to the formation of strand-overhangs on the ends of the cleaved dsDNA molecule.

As used herein "nucleic acid" shall mean any nucleic acid molecule, including, without limitation, DNA, RNA and hybrids or modified variants thereof. An "exogenous nucleic acid" or "exogenous genetic element" relates to any nucleic acid introduced into the cell, which is not a component of the cells "original" or "natural" genome. Exogenous nucleic acids may be integrated or nonintegrated in the genetic material of the target cell, or relate to stably transduced nucleic acids.

Gene therapy refers to a therapy aiming to produce a therapeutic effect through the manipulation of gene expression, genetic sequences or generally through altering the biological properties of living cells. Gene therapies have been suggested for the treatment of genetic diseases, comprising the replacement of mutated or disease causing genes or gene variants by a `healthy' gene (e.g., a gene variant not causing a disease or not carrying a (disease causing) mutation).

The instant disclosure also includes kits, packages and multi-container units containing the herein described pharmaceutical compositions, active ingredients, and/or means for administering the same for use in the prevention and treatment of diseases and other conditions in mammalian subjects.

### FIGURES

The invention is further described by the following figures. These are not intended to limit the scope of the invention but represent preferred embodiments of aspects of the invention provided for greater illustration of the invention described herein.
Figure 1: Exemplary construction of BAC-based recombinant adenovirus libraries using half-site directed fragment replacement (HFR).Schematic genetic workflow of one embodiments of the invention used for tagging pIX using T2A peptide linker with ZsGreen; i) replacement of pIX HFR locus with KnR; ii) reinsertion of pIX using PCRs fragments for both pIX and ZsGreen, connected via T2A (orange); iii) Finished modified pIX-locus with T2A-linked ZsGreen pBWH-05-plX-Kan BACmid was digested using Swal overnight. Restriction endonucleases were then heat inactivated at 65 °C and the linearized BACmid and PCR inserts were assembled using NEBuilder, combining -400 ng of BACmid and -15 ng of each PCR insert. Electrocompetent *E*. *coli* NEB1Obeta (NEB) were transformed by 4 µl of dialysed BACmid assembly , and plated on Cm-containing LB-agar plates and incubated overnight. Single colonies were picked and checked for correct assembly. Verified BACmid preparations were used for CTR as described previously.

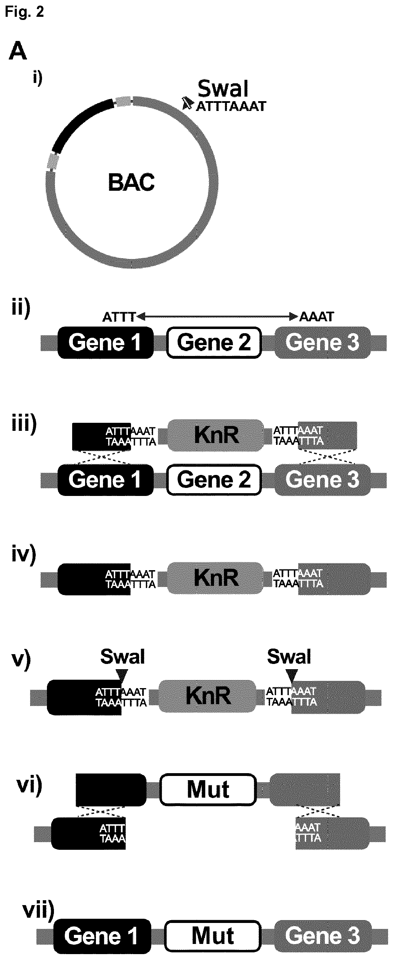

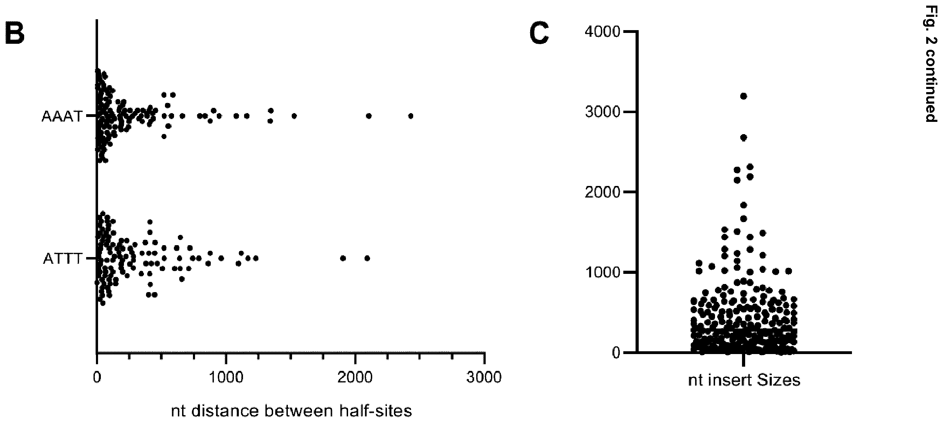

Figure 2: (A) (i) Basic cloning principle requires a BACmid containing viral genome, which is not cut by target enzyme (e.g. Swal). (ii) Insertion site is determined by selection of target region and offset half sites, (iii) red recombineering is used to replace target region with Kn resistance, (iv) completing proximal Swal half sites, (v) Intermediate construct is then digested using target enzyme, linearizing plasmid and (vi) allowing insertion of target DNA fragment, resulting in (vii) scarless modified DNA. (B) Distance in nucleotides between half-sites of Swal in exemplary BACmid containing HAdV C5delE3 genomic DNA. (C) Minimal required insert size for reassembly of BACmid (see A-vi).

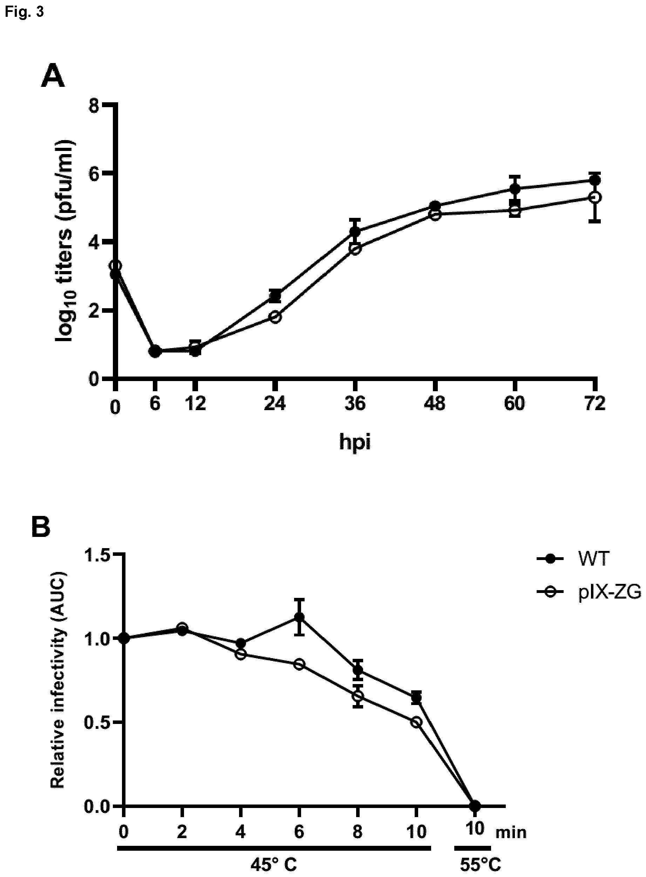

Figure 3: (A) Growth curve of recombinant human Adenoviruses, comparing HAdV C5 and HAdV C5-plXZG; (B) Thermostability experiment of recombinant human Adenoviruses, comparing HAdV C5 and HAdV C5-plXZG.

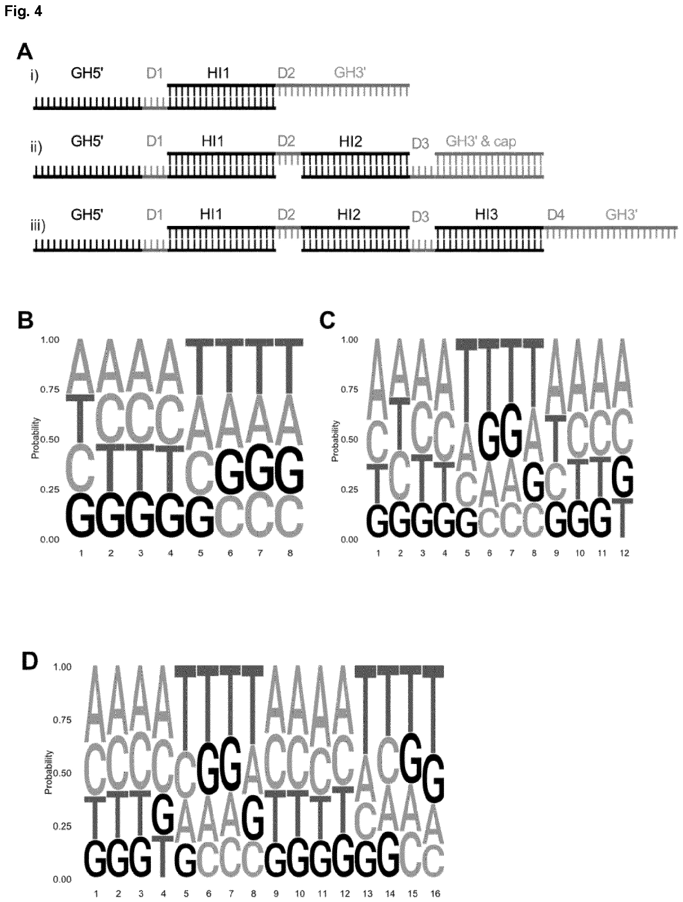

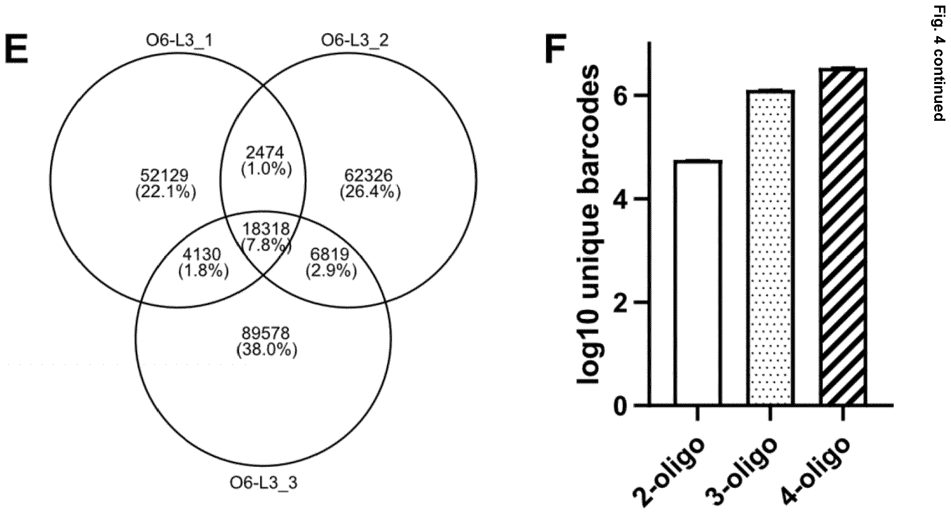

Figure 4: Barcode assembly principle and barcodes recovered from initial cloning steps in p06-based transfer vector. (A) shows basic assembly of overlaps for (i) 2-oligo approach, featuring 5' and 3' homologous regions to vector (GH5'/GH3'), 2 diversification cassettes TANNNAT (D1/02) and internal homologies for assembly (HI1). 3-oligo (ii) and 4-oligo (iii) approach follow this principle with adjusted number on diversification cassettes (131-04) and internal homologies (HI1-HI4). 3-oligo (ii) approach also features an additional capping oligo, binding to GH3' domain.
Frequency plots show frequency of assembled nucleotides in (B) 2-oligo approach, (C) 3-oligo approach and (D) 4-oligo approach. (E) Exemplary Venn diagram showcasing overlaps in identified barcodes for several •sequencing runs of the same isolation of DNA, used to calculate (F) total library diversity via capture-recapture model.
Figure 5: Barcodes recovered from secondary cloning step into BACmids. Shown is the frequency of assembled nucleotides in (A) 3-oligo libraries and (B) 4-oligo libraries.(C) Total library diversity estimate based on capture-recapture model.
Figure 6: Barcodes recovered from rescued virus. Shown is the frequency of assembled nucleotides in (A) 3-oligo libraries and (8) 4-oligo libraries.(C) Total library diversity estimate based on capture-recapture model. (D) Total viral titers of 3-oligo and 4-oligo based library virus after harvesting 4 days post transfection.
Figure 7: Overview of total barcode diversity observed in p06-based subcloning vectors, genomic virus BACmids and encapsidated DNA from virus rescue.

### EXAMPLES

The invention is further described by the following examples. These are not intended to limit the scope of the invention but represent preferred embodiments of aspects of the invention provided for greater illustration of the invention described herein.

### Materials

A list of diversified oligonucleotides, primers and plasmids used in this study can be found in the following tables.

**Table 1: Bacterial Strains**

| Strain | Features | Product ID |
|---|---|---|
| *E. coli* NEB10beta | Δ (ara-leu) 7697 araD139 fhuA AlacX74 galK16 galE15 e14-Φ80dlacZΔM15 recA1 relA1 endA1 nupG rpsL (StrR) rph spoT1 Δ(mrr-hsdRMS-mcrBC) | NEB C3020K |
| *E. coli* Pir1 | F- Δlac169 rpoS(Am) robA1 creC510 hsdR514 endA recA1 uidA(ΔM1u1)::pir-116 | Invitrogen *^{™}* C101010 |

**Table 2: Primers**

| Name | Sequence (5' - 3') | SEQ ID |
|---|---|---|
| BWHC05for | TATTGGCTTCAATCCAAAATAAGGTATATTATTGATGATGCCTCCGGGG TCCACTGCAATTACTTCTCGACCAATTCTCATGTTTGAC | 1 |
| BWHC05rev | TATTGGCTTCAATCCAAAATAAGGTATATTATTGATGATGCCTCCGGGG TCCACTGCAATTATAAACTCGACAGCGACACACTTGC | 2 |
| C5-plXKan_for | TTTGGGTAACAGGAGGGGGGTGTTCCTACCTTACCAATGCAATTTAAA TTCGTGTGGGCGG ACAATAAAGTCTTAAACTGAA | 3 |
| C5-plXKan_rev | GCAAGACACTTGCTTGATCCAAATCCAAACAGAGTCTGGTTTTTTATTT AAATTGTGGGCGGACAAAATAGTTGG | 4 |
| fC5-Kan-L_for | GAATAAGAGGAAGTGAAATCTGAATAATTTAAATTCGTGTGGGCGGAC AATAAAGTCTTAAACTGAA | 5 |
| fC5-Kan-L_rev | TTCCCACCCTTAAGCCACGCCCACACATTTAAATAAATGTGGGCGGAC AAAATAGTTGG | 6 |
| GHBfor | CCGCGTGTGTACCTCTACCTGGAGTTTTTCCCACGGTGGA | 7 |
| GHBrev | TCCACCGTGGGAAAAACTCCAGGTAGAGGTACACACGCGG | 8 |
| M13A_for | GACGGGTAAAACGACGGCCAGT | 9 |
| M13A_rev | TAATGACTCAGTACAGGAAACAGCTATGAC | 10 |
| 06-AVT for | AATGGAAGAGCTCCCATGTCAGCCGTTAAGTGTTCCTG | 11 |
| 06-AVT rev | CATTGAAGAGCTTAGAAAAACTCATCGAGCATCAAATGAAACTGCAA | 12 |
| 06-fC5_for | GCTCGATGAGTTTTTCTAAGCTCTTCAATGGAATAAGAGGAAGTGAAAT CTGAATAATTTGACGGGTAAAACGACGGCCAGT | 13 |
| 06-fC5 rev | CTTAACGGCTGACATGGGAGCTCTTCCATTTTCCCACCCTTAAGCCAC GCCCACACATTTCAGTACCATAGAGCCCACCGCATCCC | 14 |
| PCR-LibBB_ for | CATGCAAGCTTGGCGTAATCATGGTCA | 15 |
| PCR-LibBB_rev | GATCCCCGGGTACCGAGCTC | 16 |
| plXZsG_pl X_H3 | CGTCACCGCATGTGAGCAGACTTCCTCTGCCCTCTCCGGAAACCGCAT TGGGAGGGGAGGAAGCCT | 17 |
| plXZsG_pl X HS | CTACCTTACCAATGCAATTTGAGTCACACTAAGATATTGCT | 18 |
| plXZsG_ZG_ H3 | CAGAGTCTGGTTTTTTATTTATGTTTCAGGGCAAGGCGGAGCCGGAG | 19 |
| plXZsG_ZG_ HS | TCTGCTCACATGCGGTGACGTGGAGGAGAATCCCGGGCCAGCCCAGT CCAAGCACGGCCTGAC | 20 |

**Table 3: Oligonucleotides used for diversification. Underlining indicates diversification cassettes with sequence TANNNAT.**

| Name | Sequence (5' -> 3') | SEQ ID |
|---|---|---|
| Lib_Frag1 | ATATGTTTTATGTATCCAGTAACCATTGTANNNNATGACTCTAGAGG ATCCCCGGGTACCGAGCTC | 21 |
| Lib_Frag2 | TACAATGGTTACTGGATACATAAAACATATNNNNTAGTCCATCGGTT GCCCAAAGTGTTAAGAT | 22 |
| Lib_Frag3 | ATTGCGGGAAACGGCCCTAGGGGTGATATANNNNATCTTAACACTT TGGGCAACCGATGGACTA | 23 |
| Lib_Frag2_ term | TACAATGGTTACTGGATACATAAAACATATNNNNTAGACCTGCAGGC ATGCAAGCTTGGCGTAATC | 24 |
| Lib_Frag3_ term | GATTACGCCAAGCTTGCATGCCTGCAGGTCTANNNNATCTTAACAC TTTGGGCAACCGATGGACTA | 25 |
| Lib_Frag4_ term | TATATCACCCCTAGGGCCGTTTCCCGCAATNNNNTAGACCTGCAGG CATGCAAGCTTGGCGTAATC | 26 |
| Lib3_Cap | GACCTGCAGGCATGCAAGCTTGGCGTAATC | 27 |

**Table 4: Plasmids**

| Plasmid Name | Size | Features | GenBank |
|---|---|---|---|
| pGPS1.1 | 4814 bp | GPS-1 Genome Priming System transposon donor; kanamycin resistance (KanR); tetracycline resistance (TetR) | 10666445 |
| p06-A5-GFP | 3457 bp | Constitutive GFP expression under human CMV promoter; HAdV C5-targeted transfer vector, carrying part of packaging domain; kanamycin resistance (KanR)³³ | |
| pKD46 | 6329 bp | red-recombineering plasmid; Arabia sugar dependent expression of lambda red phage exo, beta and gam protein; temperature sensitive; 13-lactamase expression (AmpR) | 10829079 |
| p06-fC5-GFP | 3047 bp | Constitutive GFP expression under murine CMV promoter; HAdV C5-targeted transfer vector, carrying part of packaging domain; empty multi cloning site; kanamycin resistance (KanR) | - |
| p06-fC5-Lib2- GFP | 3089 bp | Constitutive GFP expression under murine CMV promoter; HAdV CS-targeted transfer vector, carrying part of packaging domain; multi cloning site equipped ... with 2-oligo diversified barcode; kanamycin resistance (KanR) | - |
| p06-fC5-Lib3- GFP | 3123 bp | Constitutive GFP expression under murine CMV promoter; HAdV C5-targeted transfer vector, carrying part of packaging domain; multi cloning site equipped with 3-oligo diversified barcode; kanamycin resistance (KanR) | - |
| p06-fC5-Lib4- GFP | 3157 bp | Constitutive GFP expression under murine CMV promoter; HAdV C5-targeted transfer vector, carrying part of packaging domain; multi cloning site equipped with 4-oligo diversified barcode; kanamycin resistance (KanR) | - |
| pBWH-05-delE3 | 40498 bp | Genomic BACmid; carrying mutant HAdV C5 genome (HH-Ad5-VI-wt³⁴), E3-region deleted; ITRs flanked by ACT sequences; chloramphenicol resistance (CamR) | - |
| pBWH-fC5-E1 Kn | 39014 bp | Genomic BACmid; carrying HAdV C5 genome, E3-region deleted; EI-region replaced with KanR to facilitate HFR; ITRs flanked by ACT sequences; chloramphenicol resistance (CamR); kanamycin resistance (KanR) | - |
| pVfC5-Lib2 | 38946 bp | Genomic BACmid; carrying HAdV C5 genome, E1/E3-region deleted; carrying 2-oligo diversified genetic barcoding region; Expression of GFP under murine CMV promoter; ITRs flanked by ACT sequences; chloramphenicol resistance (CamR) | - |
| pVfC5-Lib3 | 38980 bp | Genomic BACmid; carrying HAdV C5 genome, E1/E3-region deleted; carrying 3-oligo diversified genetic barcoding region; Expression of GFP under murine CMV promoter; ITRs flanked by ACT sequences; chloramphenicol resistance (CamR) | - |
| pVfC5-Lib4 | 39014 bp | Genomic BACmid; carrying HAdV C5 genome, E1/E3-region deleted; carrying 4-oligo diversified genetic barcoding region; Expression of GFP under murine CMV promoter; ITRs flanked by ACT sequences; chloramphenicol resistance (CamR) | - |
| pAR-Int5-Cas9 | 6699 bp | Constitutive sgRNA expression under human U6 promoter, targeting 5' of HAdV C5 ITR sequence; Constitutive SpCas9-FLAG-NLS expression under EF1a-promoter; ampicilin resistance | - |
| pBWH-05-plX-Kan | 43300 bp | Genomic BACmid; carrying HAdV C5 genome; pIX locus replaced with KanR to facilitate HER; ITRs flanked by ACT sequences; chloramphenicol resistance (CamR); kanamycin resistance (KanR) | - |
| pBWH-05-plXZG | 43216 bp | Genomic BACmid; carrying HAdV C5 genome; pIX protein C-terminally linked to ZsGreen" via T2A self-cleaving peptide; ITRs flanked by ACT sequences; chloramphenicol resistance (CamR) | - |
| pBWH-05 | 42376 bp | Genomic BACmid; carrying HAdV C5 genome; ITRs flanked by ACT sequences; chloramphenicol resistance (CamR) | - |
| pKSB2 | 6457 bp | Single-copy BACmid backbone; chloramphenicol resistance (CamR) | - |

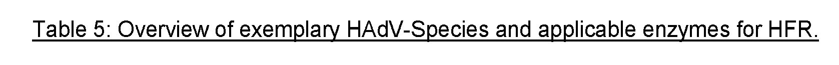

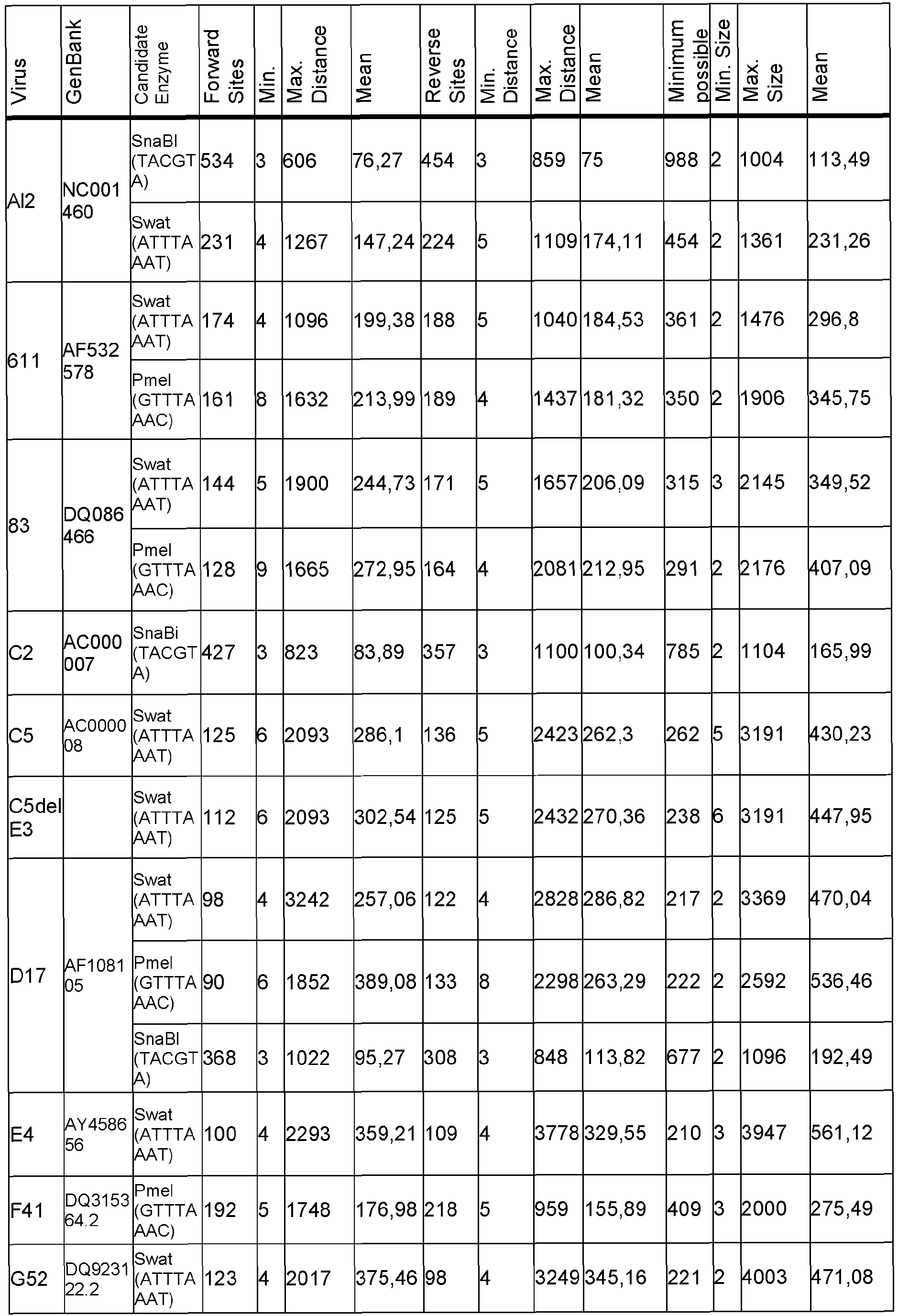

### Cell and viruses.

293A (Invitrogen) and A549 (ATCC CCL-185) cells were cultured using Dulbecco's modified Eagle's medium (DMEM) supplemented with 10% fetal calf serum (FCS) and penicillin-streptomycin (100 U/ml). The wild type HAdV-005 was cultured from inoculum provided by Albert Heim (German Adenovirus Reference Laboratory).

### Methods

### Construction of recombinant BACmids and transfer plasmids used for library assembly.

For this study, firstly CRISPR/Cas mediated terminal resolution (CTR)-permissive BACmids for replication competent and first generation recombinant human adenovirus C5 genomes were generated as previously described⁷. Amplification of pKSB2⁸ backbone via PCR was performed using primer pairs BWHC05for/GHBrev and GHBfor/BWHC05rev. Finished constructs were verified using RFLP and sequencing and verified for viability using CTR.

To allow assembly of recombinant adenoviruses using an embodiments of the present method, these BACmids were modified to allow insertion of linearized fragments using Gibson Assembly. For this, a gene cassette encoding for kanamycin (Kn) resistance was amplified via PCR on pGPS1.1 template. Primers were designed to contain 30 nt 5'-homologous to genomic regions targeted for insertion as well as 4 nt sequences completing Swal restriction enzyme sites (motifs) in addition to their priming sites for the Kn-cassette. For pIX-modification designated intermediates, primers C5-pIXKan_for and C5-pIXKan_rev, were used, for barcoding library designated intermediates, primers fC5-Kan-L_for and fC5-Kan-L_rev were used.

400 ng of the resulting PCR products were inserted into genomic BACmids using red recombineering as described by Datsenko and Wanner (2000)⁹. Resulting colonies were selected for both Kn and chloramphenicol (Cm) resistance and analyzed using RFLP. Correct insertion was furthermore confirmed using Sanger sequencing. Verified molecular clone were coined pBWH-05-plX-Kan and pBWH-fC5-E1Kn respectively.

In order to provide high quality linear inserts for efficient assembly into genomic BACmids, we generated optional transfer vectors for use with barcoding libraries, which could release its transgenic insert by restriction endonuclease digest. For this, synthetic dsDNA inserts containing genomic sequences between the targeted Swal half-sites as well as a multiple cloning site (MCS) at the position of the insertion of the randomized sequences were used and amplified using primers 06-fC5 Jor/06-fC5_rev. A GFP expression cassette was also added to allow the assessment of transfection efficiency and viral spread. These synthetic DNAs were then combined via the PCR-amplified vector backbone with primers 06-AVT_for/06-AVT_rev, based on p06-A5-GFP using Gibson Assembly. The p06-vector fragments were flanked with Sapl sites (target motifs), which allowed the release of the inset for the genomic constructions. Correct construction was confirmed using Sanger sequencing. Verified molecular clones were coined p05-fC5-GFP.

### Assembly of pIX-GFP linked genomic BACmids & rescue of recombinant virus.

To demonstrate the flexibility and reliability of the present system, the inventors decided to generate recombinant viruses with modified capsid protein pIX. For this, the inventors added a T2A-peptidel° linked ZsGreen" C-terminally to the pIX locus. To achieve this, both ZsGreen and the plX-intermediary removed region were amplified via PCR, with internal homologous overlaps, encoding for T2A peptide linker. Primer pair plXZsG_plX_H5/plXZsG_plX_H3 was used on wild type (wt) C5 BACmid to amplify intermediary removed region, primer pair plXZsG_ZG_H5/plXZsG_ZG_H3 was used to amplify ZsGreen fluorescent protein coding sequence from synthetic dsDNA gBlock obtained from Azenta life sciences. The workflow of removing and replacing this genetic regions is shown in figure 1.

### Characterization of pIX-ZsGreen linked recombinant adenovirus.

In order to check the viability of recombinant virus, thermostability assays were performed. The virus was incubated at 45 °C for up to 10 min as well as an aliquot at 55 °C for 10 min as described by Vellinga et. al.¹². Afterwards, the virus was titrated and AOC of titration steps was compared to untreated controls.

The inventors also performed multistep growth curve analysis to see if there was any impact of transgenic tagging on viral replication. For this, 293A cells were infected using MOI 0.1 rAd and incubated for 90 min. Zero-point sample was collected using both culture supernatant and infected cells, remaining samples for later time points were washed with PBS and fresh media was added. After, supernatants and cells were both collected at timepoints of up to 72 h post infection, lysed by 3 cycles of freezing and thawing and total titer determined by titration.

### Assembly of diversified barcoded genomic BACmids.

The assembly of barcoded genomic libraries was performed as a 2-step process. Firstly, p06-based linear fragments were generated by either restriction endonuclease digest or amplified by PCR using primers PCR-LibBB_for and PCR-LibBB_rev. Annealed oligonucleotide libraries were generated by adding 3 µl of each oligonucleotide (100 µM) to 500 µl lx 2.1 NEB-buffer followed by boiling at 100 °C for 3 minutes to finally let them associate during the cooling at room temperature. 2-4 diversified oligonucleotides were used for each libraries. General design of oligonucleotide consisted of 30 nt homologous regions on both ends facing to either adjacent oligonucleotides or vector backbone, as well as a 6 nt central diversity cassette (ATNNNNTA). Finally, 100 ng purified linear p06 vector fragments were assembled with 5 µl of annealed oligonucleotide libraries using NEBuilder according to the manufacturer instructions. The assembly mixes were then dialysed against deionised water, and competent *E*. *coil* Pir1 cells were electrotransformed by 2 µl of the dialysed assembly mixes. The transformed bacteria were struck out on Kn-containing LB-agar plates and incubated overnight. A pool of this bacteria was harvested by adding liquid LB media onto the plate, tapping it lightly and transferring the media to inoculate 100mlcultures for large scale plasmid DNA preparation of the recombinant transfer plasmid libraries.

The DNA extracted and purified from these pools was then digested using Hindi and Sapl overnight in order to release transgenic insert and remove non-library background. pBWH-fC5-E1Kn BACmid was digested using Swal overnight. Restriction endonucleases were then heat inactivated at 65 °C in both reactions and the non-purified fragments were assembled using NEBuilder, combining -400 ng of BACmid and -100 ng of diversified insert. Electrocompetent E. *coli* NEB1 Obeta (NEB) were transformed by 4 µl of dialysed .BACmid assembly , and plated on Cm-containing LB-agar plates and incubated overnight. A pool of bacteria was again collected as described above and DNA extracted followed by column purification.

### Rescue of viral libraries & extraction of viral genomes.

Cas9-mediated terminal resolution was performed as previously established⁷. 2 µg genomic BACmid and 1 µg gRNA-Int5 encoding Cas9 expressing helper plasmid were transfected into 293A cells, which were split into 1x10 cm dish 24 hours post transfection. Transfection efficiency was monitored using fluorescent microscopy. Cells were harvested 4 days post transfection. Harvested cells were resuspended in PBS and frozen using liquid nitrogen. After thawing and refreezing 2 more times, benzonase was added to remove both genomic DNA and remaining transfected BACmid. Benzonase was inactivated using 50 mM EDTA and DNA was then extracted using NEB Genomic DNA extraction kit. Aliquots of lysed cells from viral rescues were used to titrate virus obtained from primary rescue. This was achieved by utilizing a novel AAV-based replicon assay to measure TCIDSO, from which total plaque forming units (PFU) could be determined.

### Next generation sequencing.

To determine diversity of libraries, pools of extracted DNA from transfer vector, BACmid and viral genomes were sequenced via Illumina MiSeq next generation sequencing. In order to maximize reads containing regions of interest, a PCR was performed on DNA pools as template using primers M13A_for/M13a_rev, targeting only oligonucleotide diversified regions. These PCRs were then purified and 100 ng of each used for NGS.

### Data analysis and statistics.

NGS data was initially pre-processed and analyzed using Galaxy¹³. Sequencing adapters were trimmed and low quality basecalls (phred Q < 30) discarded. Reads were then mapped to a reference of the diversified genomic regions and resulting sequences were extracted. Using R, single 4-nt diverse regions were extracted and matched to each other, resulting in barcodes 8-16 nt in length, depending on amount of oligonucleotides used. Reads not providing a full barcode were discarded. Remaining barcodes were then analyzed further. Each pool was sequenced and analysed a total of 3 times. Using lists of unique barcodes from each run for a single sample, total diversity of library pool could be estimated using a heterogeneous capture-recapture model established by Chao (1987)¹⁴.

### Results

Selection of specific sites across genomes & using different types of fragment for insertion.

Among the methods for modification of genomic clones of rAd only the relatively inefficient red recombineering allows free choice of the site of mutagenesis. All the other methods, which can be performed with better efficiencies, rely on fixed sites. These sites are either determined by the genomic sequences or inserted artificially, both of which restrict the applicability of the method to either only few sites found by chance in the genome or only sites, which can be modified without harming the coding restrictions of the targeted sequences, respectively. To retain the flexibility of red recombineering, but open up the mutagenesis workflow with more efficient methodology we decided to use existing genomic features, which can be found frequently within genomic sequences to provide flexibility of targeting the manipulations. Many methodologies based on artificial entry sites utilize recognition sequences which cannot be found in the genome and insert or create them to provide the unique entry point. In this study, the inventors aimed to tailor the cloned HAdV-05 genome for Gibson Assembly, which allow *in vitro* manipulation of linear sequences. For this purpose creating recognition sequences, which cannot be found within the genomic constructs should be the method of choice. One of the commercially available restriction endonucleases Swal which recognizes an 8-nt sequence of ATTTAAAT cannot cut pBWH-fC5-delE3 (see Figure 2A). Instead of creating the 8 nt site we analysed the sequence of the pBWH-fC5-delE3 for the frequency of half sites of the Swal recognition sequence sequence (ATTT and AAAT). We can theoretically expect these sites to occur every 4^4 or 256 nucleotides. Within the analysed 40498 bp construct, 112 forward facing half sites (ATTT) and 125 backwards facing half sites (AAAT) were found within the genomic region. Average range between forward-sites was 303 nt, with values ranging between 2093 nt and 6 nt and 270 nt with a range of 2423 nt to 5 nt for backward-sites (see Figure 2B,C).

To create the Swal sites (target motifs) on the basis of these 'half sites' (partial target motifs) one needs to use one forward facing and one reverse facing site (motif) in combination. Accordingly the inventors generates a Swal site (target motifs) at both 'half sites', one at the 5' of the intended site of mutagenesis and the other at the 3' region, the intervening sequence will be lost and need to be reinserted together with the modified feature(s) if the integrity of the genome needs to be maintained. In respect to this setting, we also investigated our model BACmid in terms of required minimal re-insertion fragment sizes, which appeared to range from 6 nt to 3191 nt, with an average of 448 nt. The determined reinsertion requirements fits well for the limitation either using synthetic dsDNA fragments or PCR to directly generate the linear fragment, which is used as the insert fragment. Of course, sub-cloned fragments can also be used, if they can be seamlessly released from their plasmid form. Using the above analysed Swal half sites, the mutagenesis of the rAd genomes would consist of two steps, first completing the two Swal half sites adjacent to the site of the intended mutation by red recombination, and a secondly inserting the insertion of the intended mutation along with the wt sequences, which needs to be preserved between the targeted Swal half-sites (see Figure 2A). As existing genomic features directly determine insertion sites, we decided to coin our methodology Half-site-directed Fragment Replacement (HFR).

To illustrate the general applicability of the system, we chose to further analyse representative types of all human adenovirus species for the presence of half-sites of non-cutting enzymes. All analysed genomes were found to be suitable for our approach, with maximum insertion sizes being at -4 kb, still permitting easy insertion of DNA inserts (see supplement 4).

Using HFR as a tool to generate rAd constructs expressing GFP by tagging viral structural protein plX.

Using HFR to generate single mutants proved highly efficient, allowing for rapid validation of generated constructs. A kanamycin resistance cassette was inserted, replacing the N-terminus of E1B-55K and entirety of pIX. To facilitate HFR, this replaced fragment was amplified via PCR from wt plasmid as well as overlapping, homologous regions for a second PCR, containing ZsGreen fluorescent protein linked via T2A peptide linker¹⁰. Using these two fragments for Gibson Assembly resulted in highly efficient assembly of recombinant BACmid, verified by both RFLP and Sanger Sequencing. Recombinant virus rescued at similar efficiencies to wt viruses and showed strong green fluorescent activity under fluorescence microscope.

### Assessment of the basic parameters of the HFR based genomic libraries using unique Barcodes

To determine applicability of HFR for construction of rAd based libraries the inventors decided to test their approach using simple genomic barcoding. With this, they were able to determine total library diversity at any point of the procedure without having potential biases emerging due to fitness differences, which may appear upon diversifying any expressed genetic feature.

These barcodes were generated via the assembly of multiple synthetic short oligonucleotides, consisting of 30 nt long homology regions at either end used for assembly, flanking a central, 6-nt diversity region (ATNNNNTA) (see figure 3 A). In a first step, these would be assembled into the diversified genetic bar codes using a transfer vectors designated to aid assembly of the genomic constructs at the predetermined insertion sites within the rAd-genome. These barcoded recombinant genomes were then rescued (transferred) to rAd libraries by CTR. This enabled the analysis of the diversity changes over the whole process from the pooled transformation of bacteria carrying the synthetic transfer vectors to the resulting rAd libraries. To analyze the full diversity of any pool, the inventors applied a capture-recapture model based on multiple NGS runs on the same preparation to determine library diversity by the approach published by Chao (1987)¹⁴ (see figure 4 E).

All libraries were built based on an initial transformation of 10 ng of transfer plasmid DNA combined with pre-assembled oligonucleotide libraries (see figure 4A, i-iii). The amount of used oligonucleotides directly determined maximum variability of library, with 2-oligo approach (i) having 2 diverse insertion cassettes, each allowing a maximum diversity of 4^4. 3-oligo (ii) and 4-oligo (iii) libraries conversely contain 3 or 4 diversification cassettes. Maximum theoretical diversities then equate to 6.5x10"4 for 2-oligo, 1.6x10"7 for 3-oligo and 4.3x10^9 for 4-oligo based approaches. Using 3 di-versified oligonucleotides produced 1.27×10^6±1.01×10^4 barcodes transferred to the plasmid pool (see figure 4F).

A "capping" oligonucleotide was added here to provide protection against unin-tended 5' digestion of the right most diversified oligonucleotide (see figure 3A, panel ii) during Gib-son Assembly, which we found to greatly inhibit library efficiencies (data not shown). Notably, when checking frequency of nucleotides assembled in the pool, a slight bias towards the assembly of thymine can be seen. When instead assembling 4 diversified oligonucleotides, total diversity of the pool in-creased to 3.41x10A6t2.94x10^4 unique barcodes (see figure 4F).

To see if this approach of diversified oligonucleotides is able to capture the full scale of possible combinations, the inventors also prepared a reaction using 2 diversified oligonucleotides, greatly limiting the amount of possible combinations to a few times of 10⁴. As this limits the amount of possible combi-nations we can use it to check translation of predetermined diversities using our approach, which indeed revealed a library diversity of 5.61×10^4±1.57×10^2 unique barcodes out of a possible 6.5x10^4, resulting in -87.7% recovery (see figure 4F).

This pool of transfer vector was then digested by the endonuclease Sapl in order to release the fragment used in the HFR inserting the barcodes in the genomic BACs, which were also linearized to allow assembly of diversified insert.

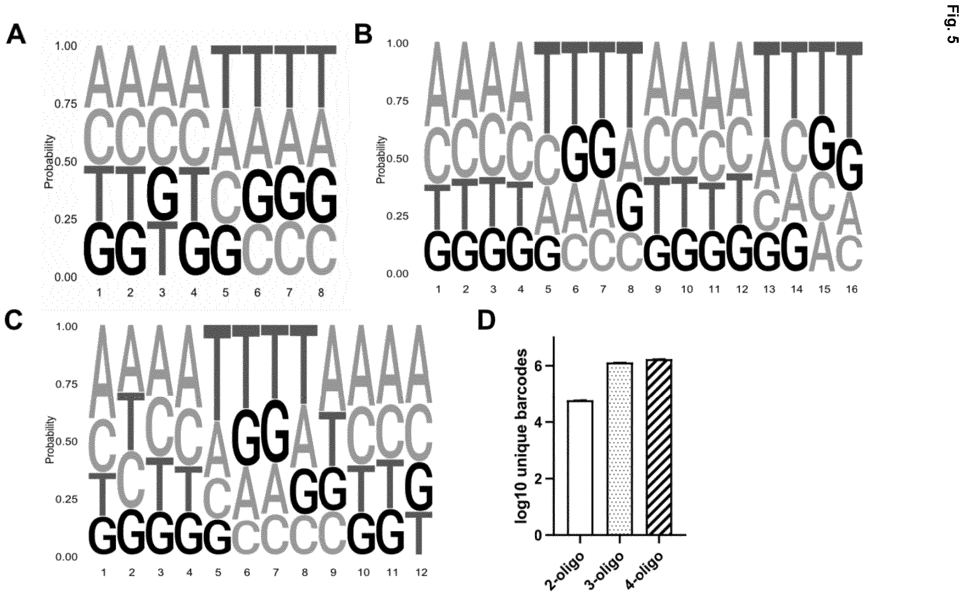

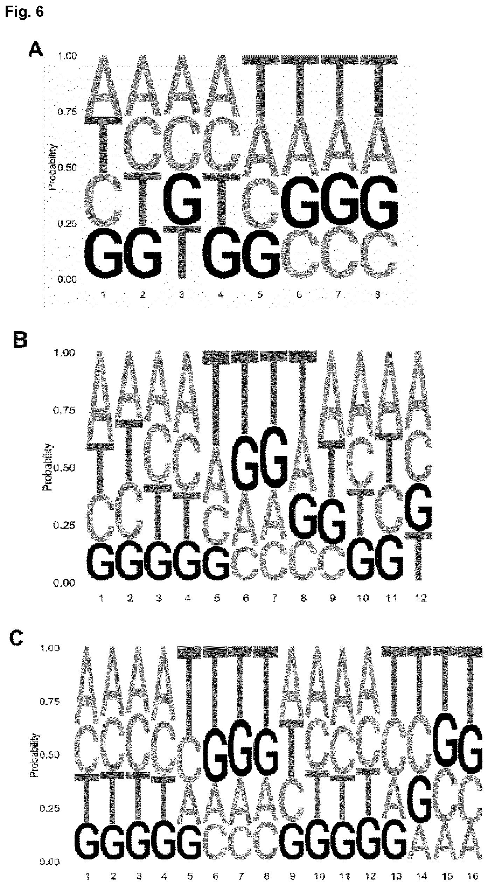

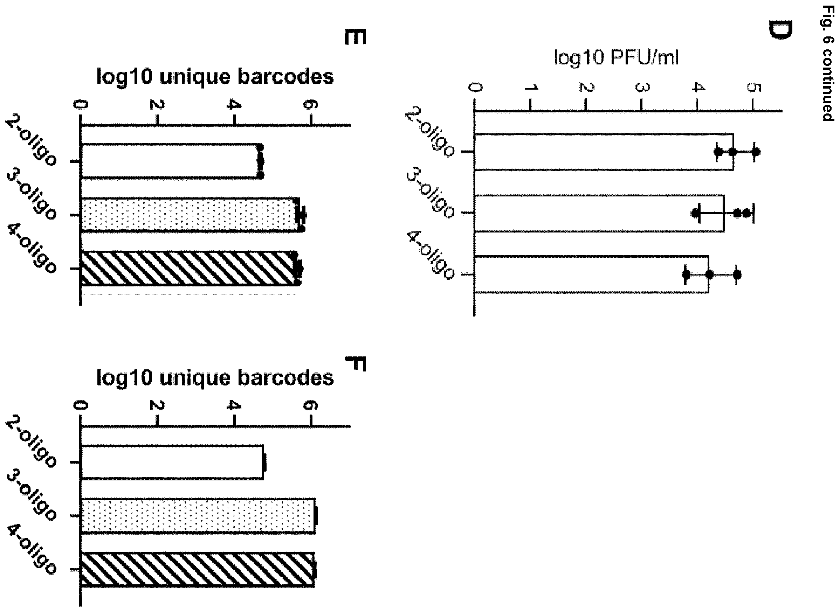

Once again analyzing the diversity of single 10 ng DNA transformations, 1.31×10^6±9.43×10^3 unique barcodes and 1.71x10^6t1.65x10^4 unique barcodes were reached at BAC level for 3- and 4-oligonucleotide diversified libraries respectively (see figure 5 D). 2-oligonucleotide diversified BACmid maintained previous diversity at 5.9×10^4±1.14×10^2 unique barcodes (see figure 5 D). Sequencing also revealed the same >99.9% correct assembly of constructs seen in previous experiments. Diversified pools of HAdV C5delE3 carrying BACmids were then rescued using CTR in 293A cells in two wells of a 6 well plate (20 cm2) using 2µg of diversified BACmid pools. Viral particles were harvested 4 days post transfection, before the emergence of visible CPE or plaques. One tenth of the samples were titrated to determine the number of resulted infections viruses. This showed on average rescue efficiencies of 5.9x10^4 PFU/ml for 2-oligo as well as 4.6×10^4 and 2.5×10^4 PFU/ml for 3-and 4-oligo based viral libraries, respectively (see figure 6 D).

The rest of the samples were processed for DNA sequencing. Non-encapsidated DNA was removed by benzonase digest before viral particles were disintegrated using proteinase K digest in presence of detergent. Resulting DNA was once again assessed for barcode diversity by NGS. Comparing multiple rescue attempts, average diversity per rescue sits at 5.23×10^5±1.01×10^5 unique barcodes per single rescue for high-diversity libraries (3-/4-oligo, see figure 6E) and 4.71×10^4±2.32×10^2 unique barcodes per single rescue for limited-diversity libraries (2-oligo, see figure 6E).

Data from 3 independent rescues from the same pool was analyzed for total diversity in order to detect any biases in rescue between experiments. This analysis revealed a total diversity of 5.84×10^4±1.25×10^2 unique barcodes for 2-oligo based libraries, 1.34×10^6±7.11×10^3 unique barcodes for 3-oligo based libraries and 1.22×10^6±5.71×10^3 unique barcodes for 4-oligo based libraries in total 60 cm^2 transfected cell culture (see figure 6F).

### Discussion

In this example, the inventors analyzed the basic features and the feasibility of library construction according to embodiments of the present invention, thereby generating recombinant adenoviruses (herein also referred to as HDFR). In embodiments this approach combines the high efficiency and flexibility of a method, such as Gibson Assembly, with the exactness and flexibility of red (recombination defective) recombination.

Handling and modifying large DNA genomes is not a trivial task^{15,16}. BAC-technology helps a lot with this¹⁷, however, many laboratories yet lack the expertise to efficiently modify these constructs. As such, currently available kits for the generation of rAd vectors are highly limited in terms of freedom when it comes to the insertion or modification into the viral genome. Here the inventors demonstrated that after combination of widely used and easy to adapt methods, directed by a new innovative target selection principle, the inventors were able to evidence a complete freedom in terms of selection of the targeted sites and the art of modification tailoring the rAd genomes. Furthermore, the present system is preferably self-controlling on several levels, using both positive and negative selection as well as differential restriction digests in order to practically completely eliminate unwanted background of mis-assembled BACmids. Cloning and modification of large BACmids is already commonly used, often in the context of recombinant viruses, attempts to use BACmid systems for the generation of feature libraries suffer from relatively low efficiency¹⁹⁻

Using the present method first as a tool to generate single recombinant variants to demonstrate its ability to precisely target coding sequences. Modification of pIX capsid genes by adding a fluorescent protein did not impact viral fitness, as expected^{12,23}, showing that there are no off-target effects on the genome, which would impede viral fitness. The present method enables in embodiments the production of recombinant adenoviral libraries, surpassing currently established methods both at flexibility and performance at least 10-fold^{24,25} while remaining easy to adapt and perform.

Using short, randomized sequences, the inventors were able to show highly efficient assembly of these barcodes in any level of the process, from cloning in transfer vector and BACmid, to the rescue of emerging virus. As these short sequences avoid any potential off-target cuts by the utilized restriction enzymes, the estimated diversity calculated from the present data supports the effectiveness of the present method. Using different amounts of randomized sequences, the maximum achievable output varies quite significantly by several orders of magnitude.

Using a lower-variability approach for assembly, limiting the amount of theoretically available barcodes below the level of maximum possible diversity, the inventors were also able to recover a vast majority of available barcodes. This is a further proof that both the prior and present method of the inventors are well fit for the full-scale recovery of genomic libraries. It is also worth mentioning that the rate of recovery is an estimate, based on interpolation of captured data using a capture/recapture model. While these models provide a good estimate of a total population size, they are not exact and usually under-estimate population sizes¹⁴. As such, actual rate of recovery is expected to be even higher than anticipated here.

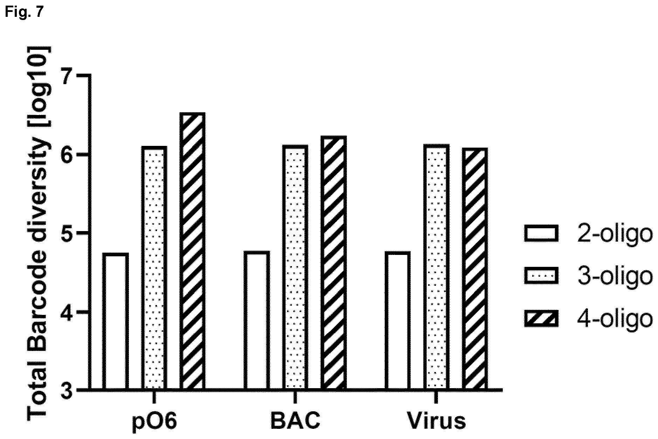

Directly comparing the levels of diversity of all 3 approaches show a clear plateau achievable with our cloning approach (see figure 7). A maximum of 7.5x10^4 unique clones per ng of transformed DNA was in this experiment the upper limit in the assembly of subcloning vectors, while for BACs the upper limit was in this example at ~1.9×10^4 unique clones per ng of transformed DNA.

Looking at viral diversity, the inventors did not expect to observe such a high amount of barcodes recovered from encapsidated DNA after rescue in a small, laboratory format. Overall diversity is significantly reduced compared to cloning beforehand, however, this was expected, if compared to previous work on the efficiency of rescue⁷. As ~2.5×10^4 unique barcodes per cm^2 of transfected cell culture in both single as well as combined experiments were obtained, the inventors expected that if overall viral diversity should ever become the limiting factor, scaling up transfection and rescue settings even further should solve this problem most likely without major loss or biasing of library diversity. What was also quite striking was the overall stability of library diversity (see Figure 7). While the higher-diversity 3- and 4-oligonucleotide based libraries lost some diversity across cloning steps, showcasing nicely the upper limit of each step, the 2-oligonucleotide based libraries maintained a consistent diversity across all steps. While the overall diversity was quite high, it is worth noting that titration revealed roughly 10-fold lower PFU counts than unique barcodes. The cause of this is most likely that either a large amount of non-infectious particles are present in these experiments or that particles aggregate and form clusters, containing many different genomes, but only registering as a single infectious unit during titration. When looking at other viral vectors currently in clinical use, mostly adeno associated virus, the inventors kmow that the major reason behind their popularity is their immense adaptability, pushed further by the ability to generate feature libraries for specific purposes. As such, the inventors wanted to create a similar technology to rAds. Unexpectedly, even when using the lowest estimates found in this study, the inventors were are able to achieve higher diversity than previously reported and very comparable levels to currently used AAV-technology⁶. Using the same principles of feature-oriented mutagenesis utilized for AAV, it can be concluded that rAds are to be used in a similar fashion, while providing some advantages and other perspectives over currently used AAV-based vectors. Current applications trying to use *in vitro* evolution based mutations suffer from low efficiency of phage-selected peptides in the rAd capsid structure²⁶.

Even classically high-diversity applications, such as phage-display libraries used for identification of immunoglobulin binding could be adapted into rAd-libraries, allowing eukaryotic expression of target protein²⁷⁻²⁹. This would help both speed and applicability of the screening platform to allow faster advances in both research and medical purposes. Lentiviral libraries are already used in similar applications³⁰, however rAd provides distinct advantages over this system as well, such as a broader tropism, allowing for wider phenotypic screenings and monogenomic transduction in comparison to lentiviral libraries^{31,32}. In summary, the present methods according to the invention allow rAd-based libraries to finally be accessible and applicable to a broad range of applications, advancing both development of therapeutics and research purposes immensely.

### REFERENCES

1. Zhao, Z., Anselmo, A. C. & Mitragotri, S. Viral vector-based gene therapies in the clinic. Bioeng. Trans!. Med. 7, (2022).
2. Boorjian, S. A. et al. Intravesical nadofaragene firadenovec gene therapy for BCG-unresponsive non-muscle-invasive bladder cancer: a single-arm, open-label, repeat-dose clinical trial. Lancet. Oncol. 22, 107-117 (2021).
3. Weinmann, J. et al. Identification of a myotropic AAV by massively parallel in vivo evaluation of barcoded capsid variants. Nat. Commun. 11, 5432 (2020).
4. Maiakovska, 0., Baumgartl, C. & Grimm, D. Determination of AAV properties by single amino acids: Go(o)d is in the details. Mol. Ther. Methods Clin. Dev. 27, 93 (2022).
5. El Andari, J. et al. Semirational bioengineering of AAV vectors with increased potency and specificity for systemic gene therapy of muscle disorders. Sci. Adv. 8, (2022).
6. Becker, J., Fakhiri, J. & Grimm, D. Fantastic AAV Gene Therapy Vectors and How to Find Them-Random Diversification, Rational Design and Machine Learning. Pathogens 11, 756 (2022).
7. Riedl, A., Fischer, J., Burgert, H.-G. & Ruzsics, Z. Rescue of Recombinant Adenoviruses by CRISPR/Cas-Mediated in vivo Terminal Resolution. Front. Microbiol. 13, 495 (2022).
8. Ruzsics, Z. et al. Transposon-Assisted Cloning and Traceless Mutagenesis of Adenoviruses: Development of a Novel Vector Based on Species D. J. Virol. 80, 81008113 (2006).
9. Datsenko, K. A. & Wanner, B. L. One-step inactivation of chromosomal genes in Escherichia coli K-12 using PCR products. Proc. Natl. Acad. Sci. U. S. A. 97, 6640-6645 (2000).
10. Liu, Z. et al. Systematic comparison of 2A peptides for cloning multi-genes in a polycistronic vector. Sci. Reports 2017 71 7, 1-9 (2017).
11. Matz, M. V. et al. Fluorescent proteins from nonbioluminescent Anthozoa species. Nat. Biotechnol. 1999 1710 17, 969-973 (1999).
12. Vellinga, J., van den Wollenberg, D. J. M., van der Heijdt, S., Rabelink, M. J. W. E. & Hoeben, R. C. The Coiled-Coil Domain of the Adenovirus Type 5 Protein IX Is Dispensable for Capsid Incorporation and Thermostability. J. Virol. 79, 3206-3210 (2005).
13. Afgan, E. et al. The Galaxy platform for accessible, reproducible and collaborative biomedical analyses: 2022 update. Nucleic Acids Res. 50, W345-W351 (2022).
14. Chao, A. Estimating the Population Size for Capture-Recapture Data with Unequal Catchability. Biometrics 43, 783 (1987).
15. Miciak, J. J., Hirshberg, J. & Bunz, F. Seamless assembly of recombinant adenoviral genomes from high-copy plasmids. PLoS One 13, e0199563 (2018).
16. Ni, N. et al. A one-step construction of adenovirus (OSCA) system using the Gibson DNA Assembly technology. Mol. Ther. - Oncolytics 23, 602-611 (2021).
17. Ruzsics, Z., Lemnitzer, F. & Thirion, C. Engineering adenovirus genome by bacterial artificial chromosome (BAC) technology. Methods Mol. Biol. 1089, 143-158 (2014).
18. Luo, J. et al. A protocol for rapid generation of recombinant adenoviruses using the AdEasy system. Nat. Protoc. 2, 1236-1247 (2007).
19. Hobom, U., Brune, W., Messerle, M., Hahn, G. & Koszinowski, U. H. Fast Screening Procedures for Random Transposon Libraries of Cloned Herpesvirus Genomes: Mutational Analysis of Human Cytomegalovirus Envelope Glycoprotein Genes. J. Virol. 74, 7720-7729 (2000).
20. Chiu, Y. F. et al. A comprehensive library of mutations of Epstein-Barr virus. J. Gen. Virol. 88, 2463-2472 (2007).
21. Osoegawa, K. & de Jong, P. J. BAC library construction. Methods Mol. Biol. 255, 1-46 (2004).
22. Kanda, T., Yajima, M., Ahsan, N., Tanaka, M. & Takada, K. Production of High-Titer Epstein-Barr Virus Recombinants Derived from Akata Cells by Using a Bacterial Artificial Chromosome System. J. Virol. 78, 7004 (2004).
23. Colby, W. W. & Shenk, T. Adenovirus type 5 virions can be assembled in vivo in the ab-sence of detectable polypeptide IX. J. Virol. 39, 977 (1981).
24. Lupold, S. E., Kudrolli, T. A., Chowdhury, W. H., Wu, P. & Rodriguez, R. A novel method for generating and screening peptides and libraries displayed on adenovirus fiber. Nucleic Acids Res. 35, e138-e138 (2007).
25. Hillgenberg, M., Hofmann, C., Stadler, H. & Löser, P. High-Efficiency System for the Construction of Adenovirus Vectors and Its Application to the Generation of Representative Adenovirus-Based cDNA Expression Libraries. J. Virol. 80, 5435-5450 (2006).
26. See Hong, S. & Boulanger, P. Protein ligands of the human adenovirus type 2 outer capsid identified by biopanning of a phage-displayed peptide library on separate domains of wildtype and mutant penton capsomers. EMBO J. 14, 4714-4727 (1995).
27. Alfaleh, M. A. et al. Phage Display Derived Monoclonal Antibodies: From Bench to Bedside. Front. Immunol. 11, 1986 (2020).
28. Hammers, C. M. & Stanley, J. R. Antibody Phage Display: Technique and Applications. J. Invest. Dermatol. 134, e17 (2014).
29. Bashir, S. & Paeshuyse, J. Construction of Antibody Phage Libraries and Their Application in Veterinary Immunovirology. Antibodies 9, 21 (2020).
30. Schmidt, F. I. et al. Phenotypic lentivirus screens to identify functional single domain antibodies. doi:10.1038/nmicrobio1.2016.80
31. Sharon, D. & Kamen, A. Advancements in the design and scalable production of viral gene transfer vectors. Biotechnol. Bioeng. 115, 25-40 (2018).
32. Bulcha, J. T., Wang, Y., Ma, H., Tai, P. W. L. & Gao, G. Viral vector platforms within the gene therapy landscape. Signal Transduct. Target. Ther. 6, 53 (2021).
33. Wodrich, H. et al. A Capsid-Encoded PPxY-Motif Facilitates Adenovirus Entry. PLoS Pathog. 6, e1000808 (2010).
34. Schreiner, S. et al. Transcriptional Activation of the Adenoviral Genome Is Mediated by Capsid Protein VI. PLoS Pathog. 8, e1002549 (2012).
35. Ruzsics, Z., Lemnitzer, F. & Thirion, C. Engineering adenovirus genome by bacterial artificial chromosome (BAC) technology. Methods Mol. Bio/. 1089, 143-158 (2014).
36. Miciak, J. J., Hirshberg, J. & Bunz, F. Seamless assembly of recombinant adenoviral genomes from high-copy plasmids. PLoS One 13, e0199563 {2018).
37. Ni, N. et al. A one-step construction of adenovirus (OSCA) system using the Gibson DNA Assembly technology. Mol. Ther. - Oncolytics 23, 602-611 (2021).
38. Riedl, A., Fischer, J., Burgert, H.-G. & Ruzsics, Z. Rescue of Recombinant Adenoviruses by CRISPR/Cas-Mediated in vivo Terminal Resolution. Front. Microbiol. 13, 495 (2022).
39. Hillgenberg, M., Hofmann, C., Stadler, H. & Löser, P. High-Efficiency System for the . Construction of Adenovirus Vectors and Its Application to the Generation of Representative Adenovirus-Based cDNA Expression Libraries. J. Viral. 80, 5435-5450 (2006).
40. See Hang, S. & Boulanger, P. Protein ligands of the human adenovirus type 2 outer capsid identified by biopanning of a phage-displayed peptide library on separate domains of wildtype and mutant penton capsomers. EMBO J. 14, 4714-4727 (1995).
41. Alfaleh, M. A. et a/. Phage Display Derived Monoclonal Antibodies: From Bench to Bedside. Front. Immunol. 11, 1986 (2020).
42. Hammers, C. M. & Stanley, J. R. Antibody Phage Display: Technique and Applications. J. Invest. Dermata/. 134, e17 (2014).
43. Bashir, S. & Paeshuyse, J. Construction of Antibody Phage Libraries and Their Application in Veterinary Immunovirology. Antibodies 9, 21 (2020).
44. Sharon, D. & Kamen, A. Advancements in the design and scalable production of viral gene transfer vectors. Biotechno/; Bioeng. 115, 25-40 (2018).
45. Bulcha, J. T., Wang, Y., Ma, H., Tai, P. W. L. & Gao, G. Viral vector platforms within the gene therapy landscape. Signal Transduct. Target. Ther. 6, 53 (2021).

## Claims

1. A method of constructing a nucleic acid molecule of interest comprising
a. Providing a circular nucleic acid molecule comprising a cloning site of interest,
b. Determining the presence of partial target motifs for at least one sequence specific endonuclease flanking the cloning site of interest (up- and downstream thereof), wherein said partial target motifs respectively comprise only a fraction, preferably essentially half, of the nucleic acid sequence of the complete target motif for the at least one sequence specific endonuclease, and wherein the circular nucleic acid molecule does not comprise the complete target motif for the at least one sequence specific endonuclease,
c. Modifying the nucleic acid molecule by inserting at the cloning site of interest a nucleic acid sequence comprising a (antibiotic) resistance gene through homologous recombination, wherein the inserted (antibiotic) resistance gene is flanked (up- and downstream) by nucleic acid sequences each comprising a sequence completing the respective partial target motif of the at least one sequence specific endonuclease determined in step b., thereby generating a modified nucleic acid molecule comprising two complete target motifs of the at least one sequence specific endonuclease,
d. Optionally selecting and/or amplifying the nucleic acid molecule modified in step c. in a microorganism, wherein the microorganism (comprising the nucleic acid molecule) is cultivated in the presence of the (antibiotic) substance corresponding to the (antibiotic) resistance gene inserted into the nucleic acid molecule in step c.,
e. Digesting the modified nucleic acid molecule with the at least one sequence specific endonuclease, thereby i) deleting the (antibiotic) resistance gene from the nucleic acid molecule and ii) linearizing the nucleic acid molecule,
f. Constructing the nucleic acid molecule of interest by inserting a nucleic acid sequence of interest at the cloning site of interest into the nucleic acid molecule linearized in step e., preferably using an *in vitro* fragment assembly technique, thereby re-circularizing the nucleic acid molecule and preferably restoring the sequence of the partial target motifs determined in step b.

2. The method according to claim 1, wherein the nucleic acid is DNA.

3. The method according to claim 1 or 2, wherein the sequence of interest is selected from (synthetic) linear DNA, a PCR fragment, a cDNA sequence generated from gRNA and a linearized cloning vector.

4. The method according to any one of the preceding claims, wherein the circular nucleic acid molecule in step a. and/or the inserted nucleic acid molecule of interest is step f. is/are recombinant adenovirus (rAd) vector(s) or adenoviral genome(s), respectively.

5. The method according to any one of the preceding claims, wherein the cloning site of interest of the circular nucleic acid molecule in step a. comprises a sequence to be deleted, and wherein the sequence to be deleted is in step c. deleted by inserting a nucleic acid sequence comprising a (antibiotic) resistance into the at the cloning site of interest.

6. The method according to any one of the preceding claims, wherein the sequence of interest comprises at least one gene of interest, e.g., a gene comprising a mutation of interest or a therapeutic gene.

7. The method according to any one of the preceding claims, wherein the sequence of interest comprises a nucleic acid sequence encoding a viral capsid or envelope, or fragments or components thereof.

8. The method according to any one of the preceding claims, wherein the sequence of interest comprises a nucleic acid sequence encoding an antibody or fragment thereof.

9. The method according to any one of the preceding claims, wherein the sequence of interest comprises a nucleic acid sequence encoding a Cas9 nuclease and/or cDNA encoding gRNA.

10. A circular nucleic acid molecule constructed according to the method of any one of the preceding claims.

11. The circular nucleic acid molecule according to claim 10, wherein the circular nucleic acid molecule is or comprises a recombinant adenovirus (rAd) vector and/or a bacterial artificial chromosome (BAC).

12. Use of the circular nucleic acid molecule according to claim 10 or 11 in a high-throughput screening approach.

13. The circular nucleic acid molecule for use according to claim 12, wherein the high-throughput screening approach comprises a screening of rAd-based gRNA libraries, a gene-expression-based screening of (monoclonal) antibodies, an *in vitro* evolution-based screening of viral particles, a screening of enzyme variants for *in vitro* evolution for improved or modified activities, a screening of random poly- or oligopeptides regarding their inhibitory effect on cellular functions, a screening of DNA sequence libraries for selecting artificial (tissue/tumor specific) enhancer elements, or a screening of randomized siRNA libraries.

14. The circular nucleic acid molecule according to claims 10 or 11 for use in the treatment of a genetic disease in a subject, wherein the treatment is a gene therapy-based treatment.

15. A pharmaceutical composition comprising the circular nucleic acid molecule according to claims 10 or 11 for use in the treatment of a genetic disease in a subject, wherein the treatment is a gene therapy-based treatment.

16. An immunogenic composition, such as a vaccine, comprising the circular nucleic acid molecule according to claims 10 or 11.

17. The immunogenic composition according to claim 16 for use in the induction of an immune response (vaccination reaction) in a subject.
